# EUROPEAN PATENT SPECIFICATION

(11) **EP 2 282 746 B1**
(45) Date of publication and mention of the grant of the patent: **28.10.2015**
(21) Application number: 09767153.1
(22) Date of filing: 27.03.2009
(51) Int. Cl.: A61L 24/00, A61L 24/10

(54) **COMPOSITIONS AND METHODS USING STEM CELLS IN CUTANEOUS WOUND HEALING**
ZUSAMMENSETZUNGEN UND VERFAHREN UNTER VERWENDUNG VON STAMMZELLEN ZUR WUNDHEILUNG AUF DER HAUT
COMPOSITIONS ET MÉTHODES UTILISANT DES CELLULES SOUCHES DANS LA CICATRISATION DE PLAIES CUTANÉES

(30) Priority: 27.03.2008 US 39941 P
(43) Date of publication of application: 16.02.2011
(73) Proprietor: Neostem, Inc, New York, NY 10170 (US)
(72) Inventor: FALANGA, Vincent, Boston, MA 02115 (US)
(74) Representative: Pohlman, Sandra M.
(86) International application number: PCT/US2009/038666
(87) International publication number: WO 2009/154840

(56) References cited:
- EP-A1- 1 900 370
- US-A1- 2004 191 225
- US-A1- 2006 095 075
- US-A1- 2007 286 881
- R.W.E PIKAART: "Human Fibrin as a Cell Carrier for Heart Valve Tissue Engineering", January 2008 (2008-01), Thesis from Eindhoven Universoty of Technology, Eindhoven University of Technology, XP002700008, pages 1-x, * page 6, paragraph 2.1.1 - page 7, paragraph 2.1.3 * * page 11, paragraph 3.1.1 - page 14, paragraph 3.2.3 *
- O'LEARY R ET AL.: 'A novel in vitro dermal wound-healing model incorporating a response to mechanical wounding and repopulation of a fibrin provisional matrix.' IN VITRO CELL DEV BIOL ANIM. vol. 39, no. 5-6, May 2003, pages 204 - 207, XP008051297
- KUCIA M ET AL.: 'Physiological and pathological consequences of identification of very small embryonic like (VSEL) stem cells in adult bone marrow.' J. PHYSIOL. PHARMACOL. vol. 57, no. SUP.5, November 2006, pages 5 - 18, XP002604058
- GORODETSKY R ET AL.: 'Haptotactic and growth stimulatory effects of fibrin(ogen) and thrombin on cultured fibroblasts.' J LAB CLIN MED. vol. 131, no. 3, March 1998, pages 269 - 280, XP009046579

## Description

### FIELD OF THE INVENTION

This application relates to compositions, uses and methods of making compositions using stem cells for wound healing applications.

### BACKGROUND

Chronic wounds remain a formidable challenge. In spite of recent advances from breakthroughs in recombinant growth factors and bioengineered skin, up to 50% of chronic wounds that have been present for more than a year remain resistant to treatment.

Fibrin sealants are a type of surgical "glue" that is made from human blood-clotting proteins, and are typically used during surgery to control bleeding. Fibrin sealants have been used to augment hemostasis, seal tissues, facilitate targeted delivery of drugs, and in treatments of wounds. See *e.g*., U.S. Patent Publication Nos. 2008/0181879, 2008/0199513, 2004/0229333, and 2006/0240555. EP1900370 discloses a fibrin glue composition for topical application to neural tissue, the composition optionally comprising bone marrow stem cells. The fibrin glue is obtained by mixing fibrinogen in the range of 10-1000 mg/ml and aprotinin in the range of 10-500 KIU/ml and calcium chloride in the range of from 1 mM to 100 mM. The preferred concentration of fibrogen is 100 mg/ml.
Pikaart RWE, January 2008 (Thesis from Eindhoven University of Technology) discloses a human fibrin disk model comprising 5 to 15 mg/ml fibrinogen with human vena saphena (i.e., vein) cells for heart valve tissue engineering. While there have been advances in the treatment of difficult-to-heal wounds in the last few years, there is still a considerable percentage (up to 50%) of chronic wounds, particularly those that are of more than 1 year in duration, that remain unresponsive to advanced treatment approaches. Thus, there remains a need to stimulate the healing of acute and chronic wounds to a level that is not presently possible with standard care measures or recently developed innovative approaches.

### SUMMARY

The present invention provides fibrin sealant compositions, therapeutic uses thereof and methods of making fibrin sealant compositions comprising stem/progenitor cells.

In a first aspect, the present invention provides a fibrin sealant composition comprising a fibrinogen complex (FC) component, a thrombin component, and a cellular component, wherein the concentration of fibrinogen used to form the gel is between from about 2 to about 5 mg/ml, wherein the cellular component comprises one or more of stem/progenitor cells, and wherein the fibrin sealant comprises at least 1.5 - 2.0 X 10⁶ stem/progenitor cells / cm².

According to some embodiments, the fibrin sealant is produced by combining a fibrinogen complex (FC) component, thrombin component, a cellular component in admixture.

According to some embodiments, the fibrin sealant is produced by combining a fibrinogen complex (FC) component and a thrombin component in admixture. In another embodiment, the cellular component may be added to the FC component before admixture of the FC component with the thrombin component. According to some embodiments, the cellular component may be added to the thrombin component. According to some embodiments, cellular component may be added to the mixture of FC and thrombin before the components are allowed to form the fibrin gel.

According to preferred embodiments, protease inhibitors are excluded from the fibrin sealant compositions of the present invention.

The invention also provides a fibrin sealant composition for use in therapy. Such use includes administering to a patient a fibrin sealant comprising a cellular component. According to some embodiments, the fibrin sealant is in the form of a polymerized gel or spray. The fibrin sealant of the present invention may be administered to the site of the wound as needed during the course of the healing process, such as once, twice, three times, four times, five times, or more.

According to some embodiments, the invention provides a fibrin sealant composition for use in treating acute or chronic wounds. Generally, the patient is suffering from a wound which would benefit from the compositions claimed herein, which would be apparent to one of ordinary skill in the art. According to preferred embodiments, the wound is a chronic cutaneous wound.

In one embodiment, the fibrin sealant is administered to a patient using methods well-known in the art, such as injection, spray, endoscopic administration or pre-formed gel and other methods known to one of ordinary skill in the art.

Accordingly, the present invention provides a fibrin sealant composition for use in therapy, comprising: a) combining stem/progenitor cells with a fibrin sealant to form a wound sealant, said fibrin sealant comprising calcic thrombin and fibrinogen, wherein the concentration of calcic thrombin is about 25 U/ml, wherein the concentration of fibrinogen is from about 2 to about 5 mg/ml, and wherein the final concentration of stem/progenitor cells is at least from about 1.5 to about 2.0 X 10⁶ stem/progenitor cells / cm²; b) administering the fibrin sealant composition to a cutaneous wound, wherein the fibrin sealant composition is administered in the form of a polymerized gel or spray.

According to preferred embodiments, the fibrin sealant composition for use in therapy according to the invention is for use in ameliorating the formation of scars at a wound site.

According to preferred embodiments, the fibrin sealant composition for use in therapy to the invention is for use in treating scleroderma.

Also provided by the present invention is a method of making a fibrin sealant comprising: providing a fibrinogen complex (FC) component, a calcic thrombin component, and a cellular component; adding the cellular component to the FC component before admixture of the FC component with the calcic thrombin component; and adding the calcic thrombin component to the combined FC/cellular component mixture, wherein the concentration of fibrinogen is from about 2 to about 5 mg/ml, and wherein the final concentration of stem/progenitor cells is at least from about 1.5 to about 2.0 X 10⁶ stem/progenitor cells / cm².

According to preferred embodiments, the stem/progenitor cells may be selected from the group consisting of bone marrow derived cells, hematopoietic stem cells, mesenchymal stem cells, peripheral blood stem cells, and mixtures and combinations thereof. According to preferred embodiments, the stem/progenitor cells are very small embryonic-like (VSEL) stem cells. According to preferred embodiments, the VSEL stem cells are CD34⁺/lin⁻/CD45⁻ or Sca-1⁺/lin⁻/CD45⁻.

According to preferred embodiments, the concentration of calcic thrombin is about 25 U/ml.

According to preferred embodiments, the fibrin sealant is administered to the site of the wound at least two times over the span of three weeks. According to preferred embodiments, the fibrin sealant is administered to the site of the wound in the form of a spray at a CO₂ psi of less than 5 psi or 34 475 Pa (N/m²). According to preferred embodiments, the fibrin sealant is topically applied to the site of the wound. According to preferred embodiments, a skin substitute is applied to the site of the wound.

Also described herein is a kit for preparing a fibrin sealant comprising, a) a first vial or first storage container containing a fibrinogen complex component, wherein the vial optionally comprises a cellular component, and b) a second vial or second storage container having a thrombin component, said kit optionally containing a third vial or third storage container having a cellular component when said first vial or first storage container does not include a cellular component, said kit further containing instructions for use thereof. The kit may also comprise instruments for use or administration of the fibrin *sealant in vitro or in vivo.* The kit may also comprise a means for characterizing the cellular component. Such means includes reagents for identifying the presence of stem/progenitor cellular markers. Reagents for identifying the presence of stem/progenitor cellular marker include, but are not limited to, antibodies.

Also described herein is a kit for preparing a fibrin sealant comprising, a) a first vial or first storage container containing a fibrinogen complex component, and b) a second vial or second storage container having a thrombin component, wherein the vial optionally comprises a cellular component, said kit optionally containing a third vial or third storage container having a cellular component when said second vial or second storage container does not include a cellular component, said kit further containing instructions for use thereof. The kit may also comprise instruments for use or administration of the fibrin *sealant in vitro or in vivo.* The kit may also comprise a means for characterizing the cellular component.

The invention also provides a kit for preparing a fibrin sealant comprising, a) a first vial or first storage container containing a fibrinogen complex component, wherein the concentration of fibrinogen is from about 2 to about 5 mg/ml, and b) a second vial or second storage container having a thrombin component, said kit further containing instructions for use thereof. According to some embodiments, the kits of the present invention provide that the concentration of thrombin is about 25 U/ml. According to some embodiments, the kits of the present invention provide that the first vial or first storage container optionally comprises a cellular component. According to some embodiments, the kits of the present invention provide that the optional third vial or third storage container having a cellular component when said first vial or first storage container does not include a cellular component. According to some embodiments, the kits of the present invention may further comprise instruments for use or administration of the fibrin sealant *in vitro or in vivo.*

According to some embodiments, the kits of the present invention provide may further comprise reagents for characterizing the cellular component.

### BRIEF DESCRIPTION OF FIGURES

The patent or application file contains at least one drawing executed in color. Copies of the patent or patent application publication with color drawings will be provided by the Office upon request and payment of the necessary fee.

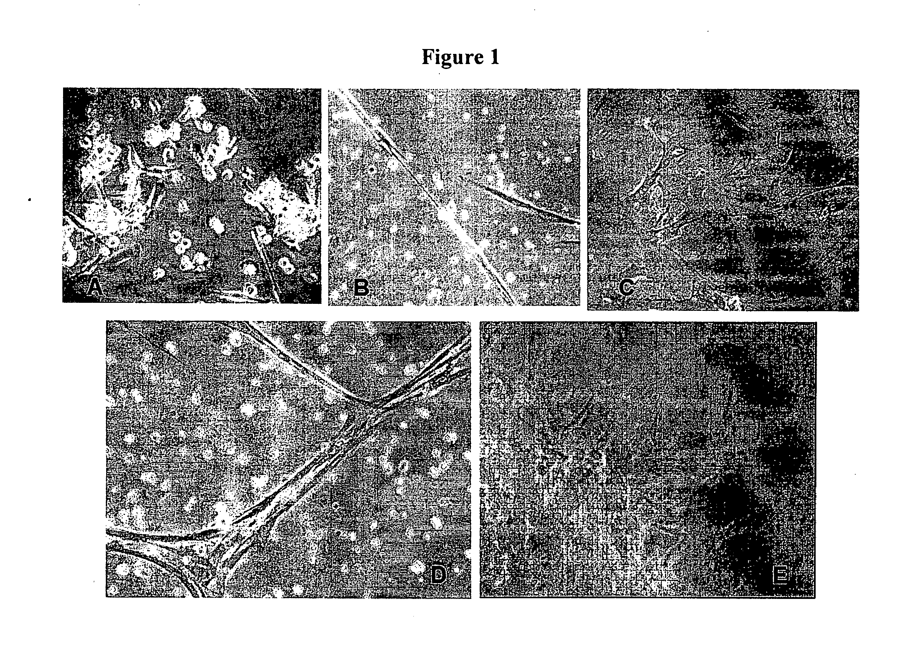

Figures 1 A-E. Morphologic appearance of bone marrow-derived cultured cells and their migration from fibrin. (A) Appearance of cultured cells on tissue culture plastic by day 2 after seeding from Ficoll separated bone marrow aspirate. A variety of morphologies is seen, ranging from round to spindle cells. (B) As early as day 5 in culture, cells began to assume a spindle shape, often with cells joined together along their long axis. (C) At later times in culture (10-12 passages), cells became larger and generally lost the spindle-shape morphology, and assumed a more unusual polygonal shape. (D) During early passage of cells grown on tissue culture plastic (up to 10 passages), one can often see extreme spindle cell morphology, with the development of islands of three-dimensional structures. (E) Migration by 4 hours of spindle-shaped bone marrow-derived cells seeded in culture as a spray of fibrin containing bone marrow-derived cells. All figures are at a magnification of 10X.

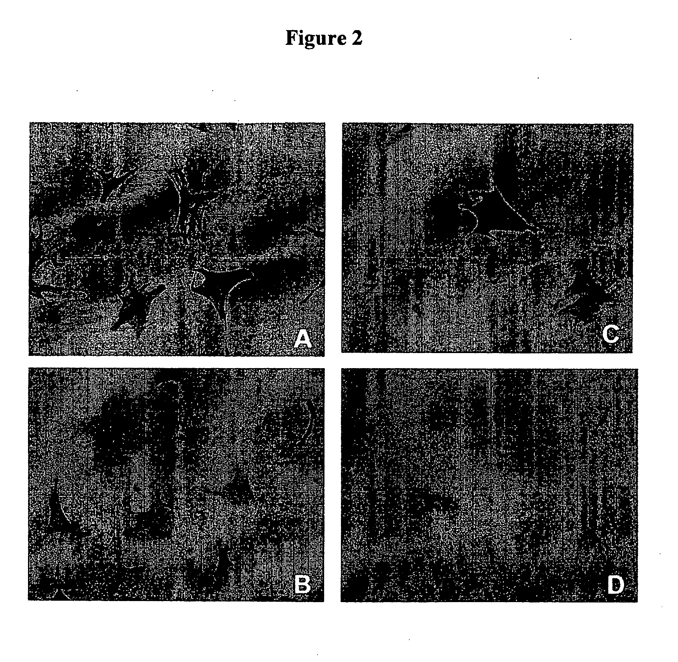

Figures 2 A-D. Immunostaining of cultured cells for selected CD markers. Bone marrow-derived cultured cells were grown on glass slides and immunostained with CD markers. These representative examples show that the cultured cells were positive for MSC markers ((A) CD29, (B) CD44, (C) CD90) and negative for hematopoietic markers ((D) CD34). Magnification 10X.

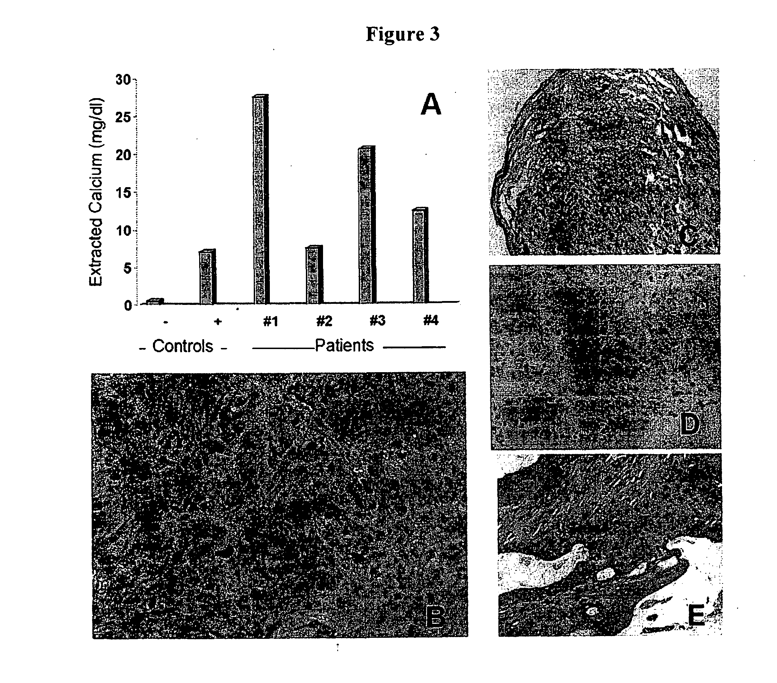

Figures 3 A-E. Functional assays for differentiation of cultured MSC. (A) Representative results of bone formation, using calcium assays, for four consecutively tested cultured cells from four different patients. Positive (established strain of MSC) and negative (human dermal fibroblasts) controls are also included in the graph. (B) Adipose tissue formation, using Oil Red O, of lipid droplets in a representative example of cultured cells; the red-staining cells indicate fat-laden cells. (C) Representative example of a pellet of cultured cells staining positive for cartilage, using Safranin O. (D) Negative control for cartilage, using human dermal fibroblasts. (E) Positive control for cartilage, using archival cartilage tissue. Magnification 10X in all cases.

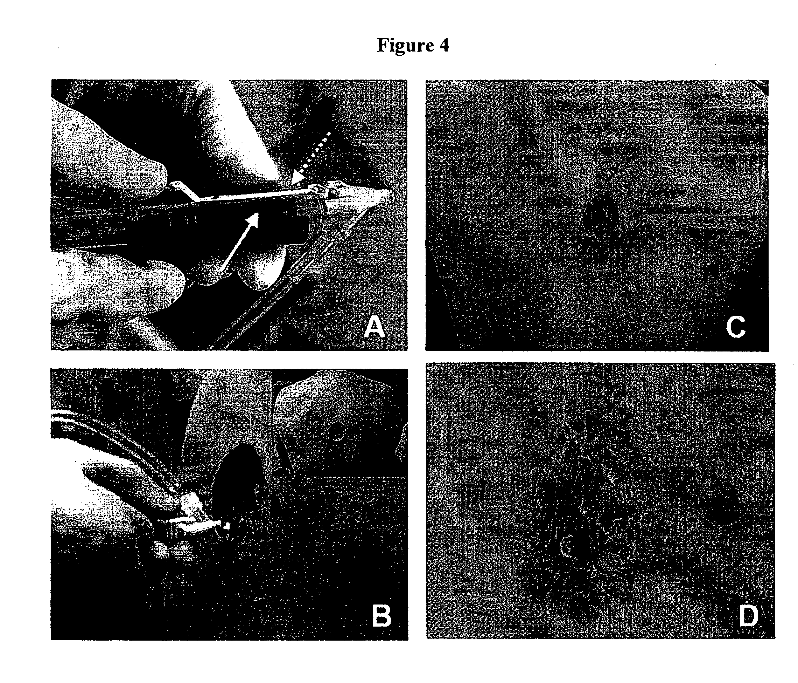

Figures 4 A-D. Application of bone marrow-derived cultured MSC to acute human wounds. (A) The cells were applied directly to the wound using a fibrin polymer spray delivered from a double barreled syringe. The arrows point to the individual barrels filled with either thrombin or the cell-containing fibrinogen solution. The tubing, attached to the common spray jet area below the syringe, was connected to CO₂. (B) Application of the cultured cells to the wound at baseline and immediately after surgery, was done by pressing on the common plunger of the double-barreled syringe shown in (A), and approximately 2 cm away from the wound bed. The inset shows the large wound on the back of the subject, who was sitting up. No run-off of the sprayed material is observed. (C) Appearance of the wound at week 6, showing complete filling of the wound bed and almost complete epithelial resurfacing. (D) Complete healing of the wound occurred by week 7, and the wound remained healing by week 12, as shown. The pink area to the right of the healed wound indicates a healed biopsy site.

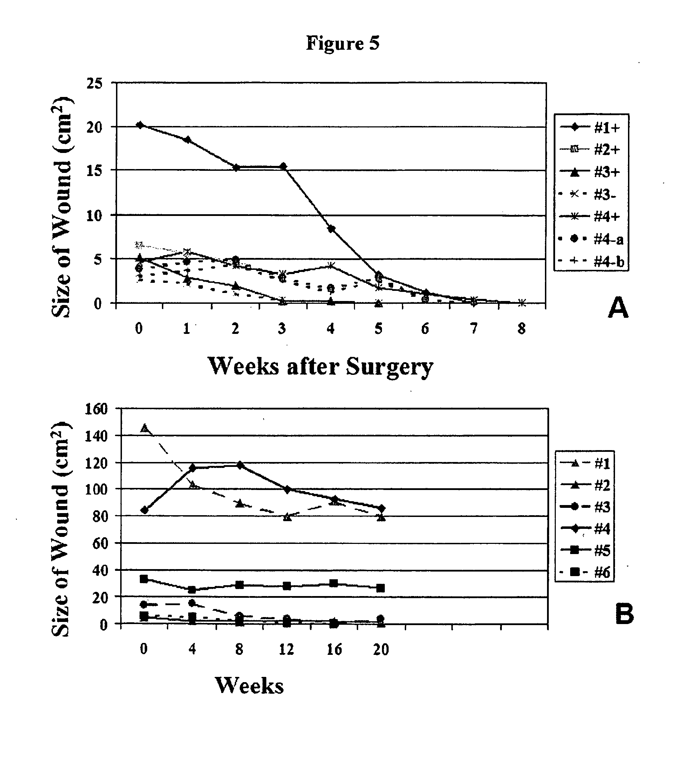

Figures 5 A-B. Rate of healing of the human wounds. The autologous bone marrow-derived cultured MSC were applied using a fibrin spray to both acute and chronic human wounds. (A) Healing trajectory of four subjects with acute wounds after removal of skin cancer. The numbers refer to the four individual patients, and "+" or "-"next to the numbers indicates wounds treated with either MSC in the fibrin spray or the fibrin spray alone, respectively. The dashed lines also represent the healing of wounds treated with fibrin alone. Patient #4 had one wound treated with cells, and two with fibrin alone (-a and -b). (B) Healing trajectory of subjects with chronic wounds all treated with MSC in a fibrin spray.

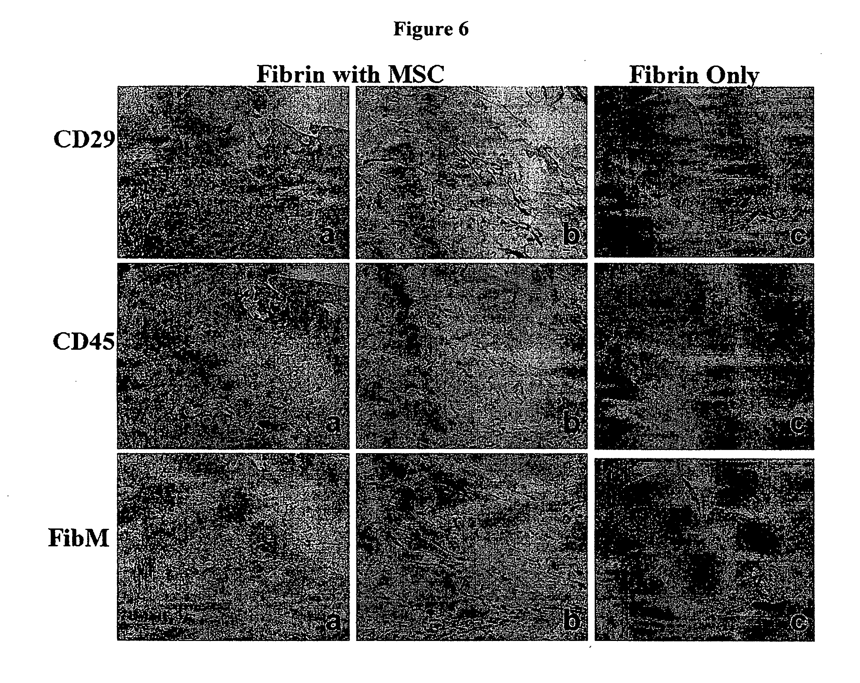

Figure 6. Immunostaining of acute wounds at day 8 after MSC application. Sequential sections of formalin-fixed biopsy specimens were immunostained for CD29, CD45, and prolyl hydroxylase as a specific marker for human fibroblasts (fibroblast marker: FibM). The "a," "b," and "c" refer to magnifications of 4x, 10x, and 2x, respectively. The blue staining is due to the marker blue ink applied to the top of the biopsy specimens. The left two columns of photomicrographs represent sequential sections of a representative wound treated with MSC in fibrin, while the right single column of photomicrographs represents a control wound treated with fibrin alone. For the MSC treated wounds, spindle-shaped cells positive for both CD29 and the fibroblast marker (FibM) are present in the superficial layers of the wound bed, which is where the cultured cells were applied. The immunostained cells appear to be distinct from those positive for CD45, the leukocyte common antigen (LCA). Conversely, as seen in the fibrin-only group, the superficial bed of the wound treated with fibrin alone is deficient in CD29 expressing cells and the cells expressing fibroblast marker (FibM). Only the deep aspect of the wounds shows immunostaining for these markers, most likely representing an endogenous and deeper cellular component.

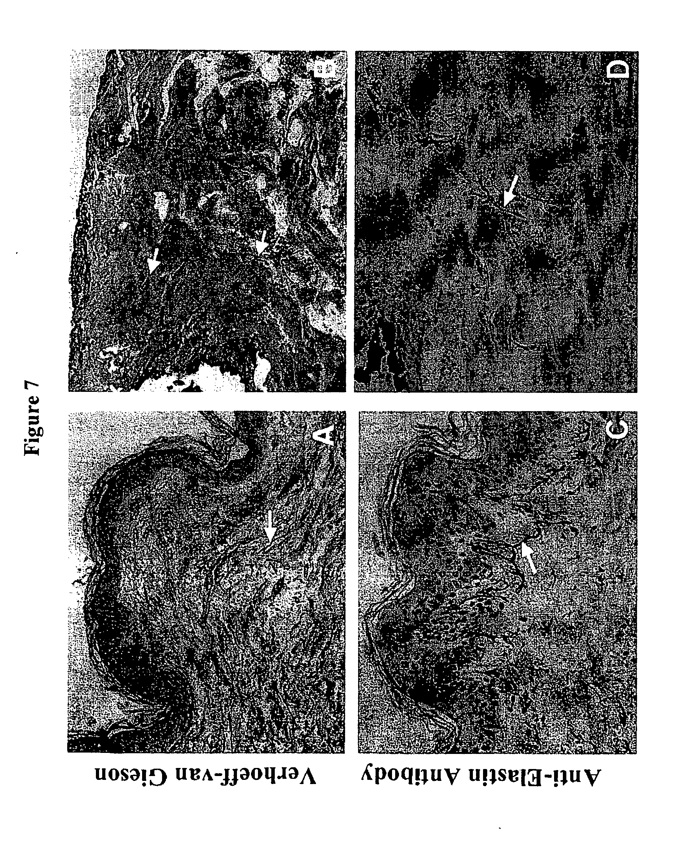

Figures 7 A-D. Elastic fibers in acute human wounds at day 8 after MSC application. The left side of the pictures indicates the two different methods of elastic fibrin staining. The Verhoeff-van Gieson stains the elastic fibers black, while the elastic fibers immunostained with a specific elastic antibody stain red. Arrows point to individual and representative elastic fibers. The left upper and lower panels (A) and (C) represent control normal skin, while the right upper and lower panels (B) and (D) are from the superficial wound bed to which the MSC had been applied. Using both stains, there appears to be definite new formation of elastic fibers. Magnification 10x.

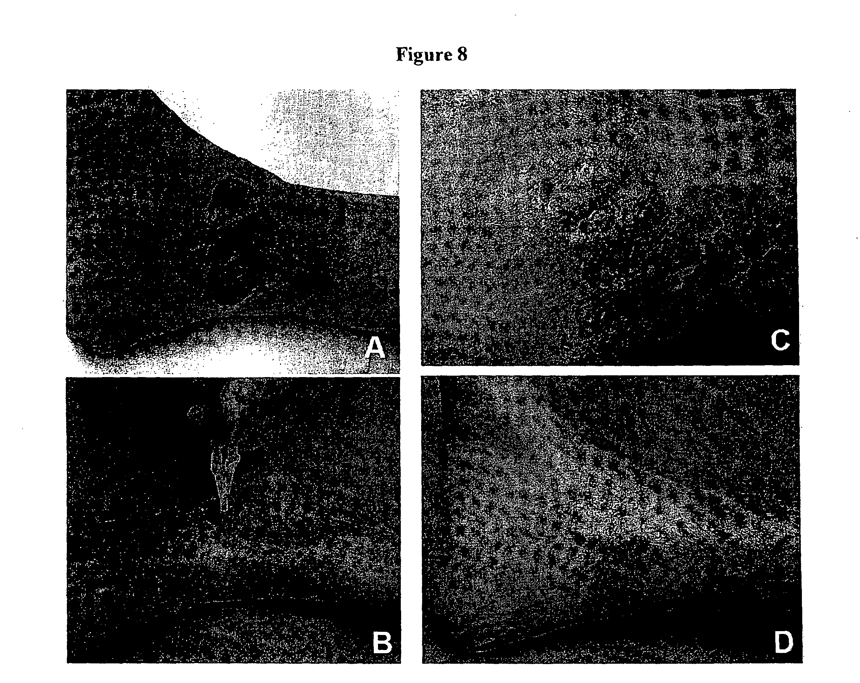

Figures 8 A-D. Application of bone marrow-derived cultured cells to human chronic wounds. (A) Nonhealing wound over the ankle of a subject at baseline; (B) Third application of MSC in a fibrin spray to the now healing wound; (C) At 3 months, the wound is almost healed. The wound bed has filled in completely, and only a small eroded surface is present. (D) The wound then went on to heal with minimal scarring. The photographs show final documentation of complete wound closure at 6 months.

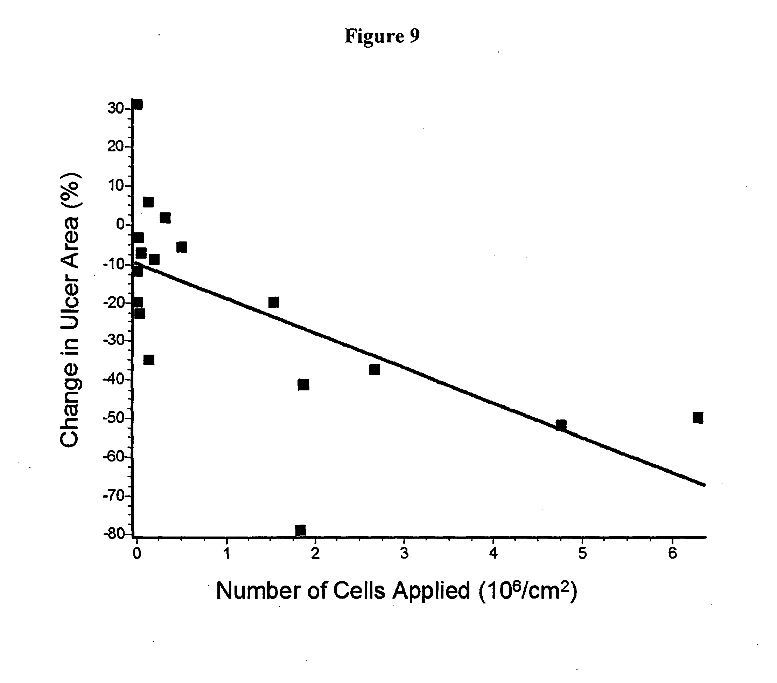

Figure 9. Correlation of wound healing with the number of MSC applied. The graph represents the correlation between the number of MSC applied to wounds in a fibrin spray and the percent change in ulcer area at 2-4 weeks after each application in patients with chronic wounds (n = 17 data points). Analysis was done using Spearmen Rank Correlation; r = -0.6389 (corrected for ties) with the 95% confidence interval of -0.8606 to -0.2135; p = 0.0058. Application of greater than 1x10⁶ cells/squared cm of the wound was highly associated with a subsequent (2-4 weeks) decrease of at least 10% in ulcer size (Fisher's Exact Test two-sided; p = 0.0345).

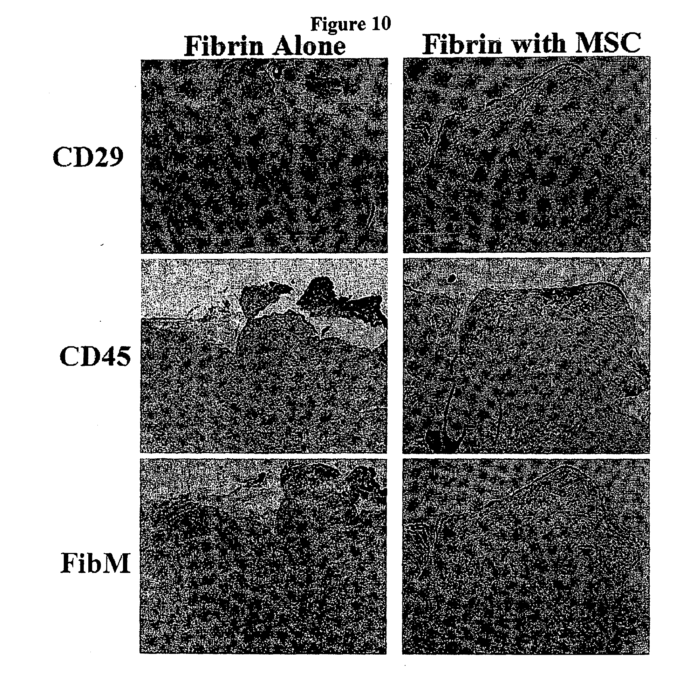

Figure 10. Immunostaining of chronic wounds before and at week 3 after MSC application. Sequential sections of formalin fixed biopsy specimens from a representative chronic wound were immunostained for CD29, CD45, and prolyl hydroxylase as a specific marker for human fibroblasts (fibroblast marker: FibM). All magnifications are 10x. The left and right panels refer to the wound treated with fibrin alone or MSC-containing fibrin spray, respectively. The blue staining is due to the marker blue ink applied to the top of the biopsy specimens. The panels show spindle shaped cells positive for both CD29 and the fibroblast marker in the superficial layers of the wound bed, where the cultured cells were applied. The immunostained cells appear to be distinct from those positive for CD45, the leukocyte common antigen (LCA).

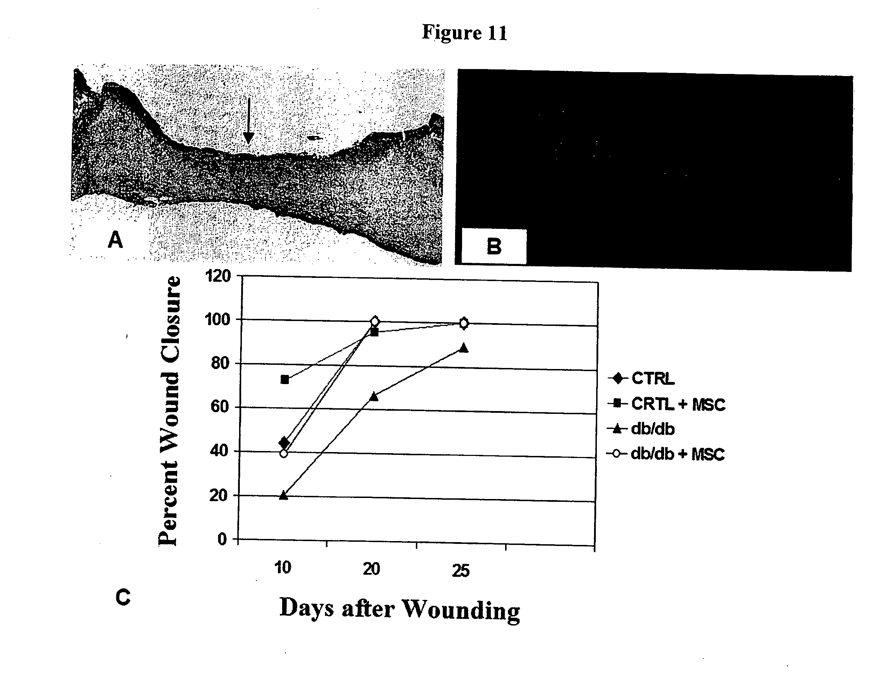

Figures 11 A-C. Histology and effect on healing in mouse wounds treated with MSC. (A) Histology of a representative mouse wound 5 days after application of syngeneic MSC in a fibrin spray. The wound bed contains a large number of cells; 4x. (B) Red fluorescently labeled MSC were applied in a fibrin spray to the mouse wounds. By day 5, the cells had migrated into the dermal component of the wound bed. The photomicrograph represents a histological section adjacent to that shown in (A); 10x. (C) The graphs represent the results of healing when treating mouse tail wounds with syngeneic MSC. Both db/db mice and their control littermates were treated either with fibrin alone or with MSC-containing fibrin spray. Each point represents the mean + SEM from four mice.

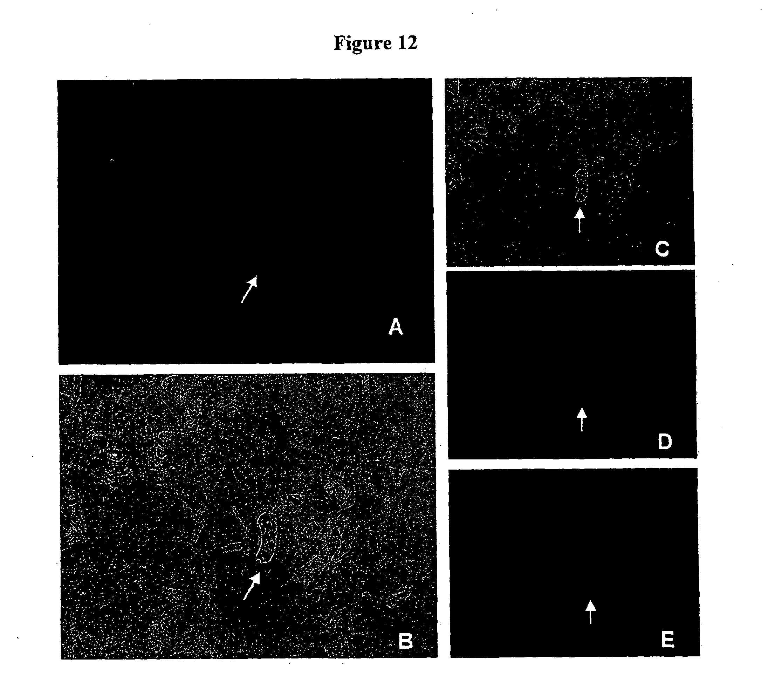

Figures 12 A-E. Identification of GFP+ MSC in mouse wounds. Photomicrographs of C57BL/6 mouse tail wounds 18 days after wounding. The tail wounds were treated with a fibrin spray containing GFP+ syngenetic bone marrow-derived cultured MSC. Frozen sections were analyzed using the Zeiss Axioplan 2 imaging system with the Axio CAM. GFP+ blood vessels (arrows) were identified by FITC (510-560nm; (A)). Adjacent sections were analyzed by filters for light microscopy (C), Texas Red (645/75 nm; (D)), and DAPI (435- 475 nm; (E)). Using these filters, a composite photomicrograph was obtained, showing the specificity of the green fluorescence (B). Magnification 200x.

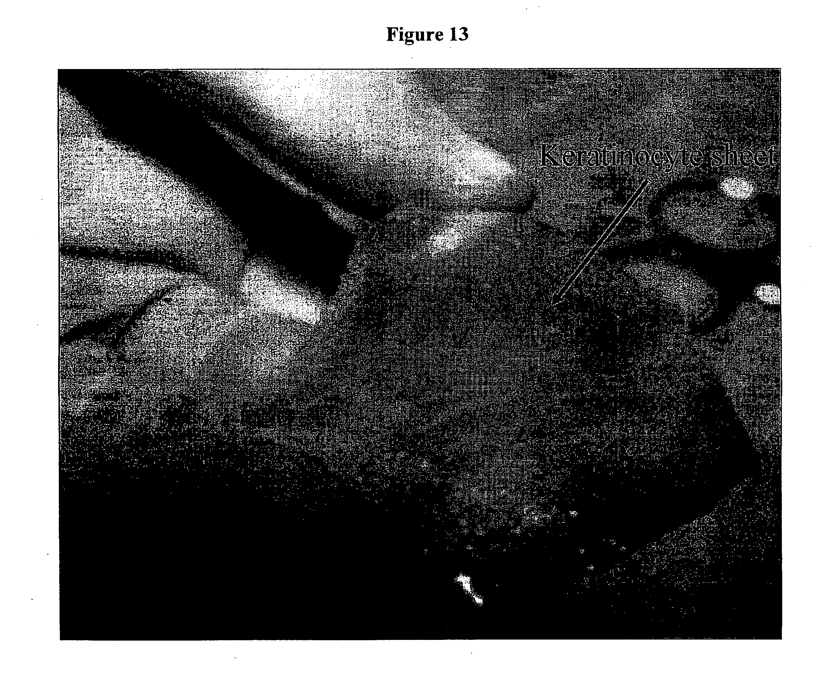

Figure 13. Example of keratinocyte sheet grown from patient's own skin.

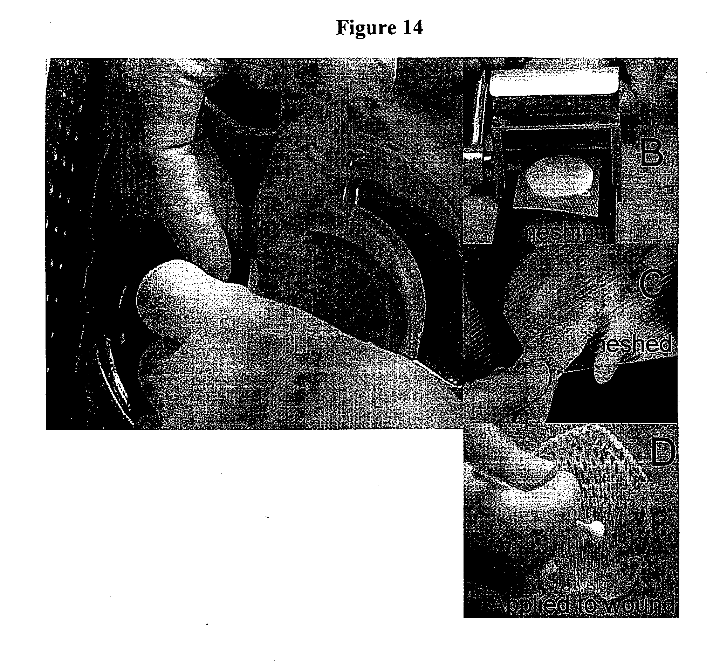

Figures 14 A - D. Illustrates examples of (A) Bioengineered skin: Living bilayered skin construct (BSC); (B) the meshing procedure; (C) the meshed fabric; and (D) application of the meshed fabric to the wound.

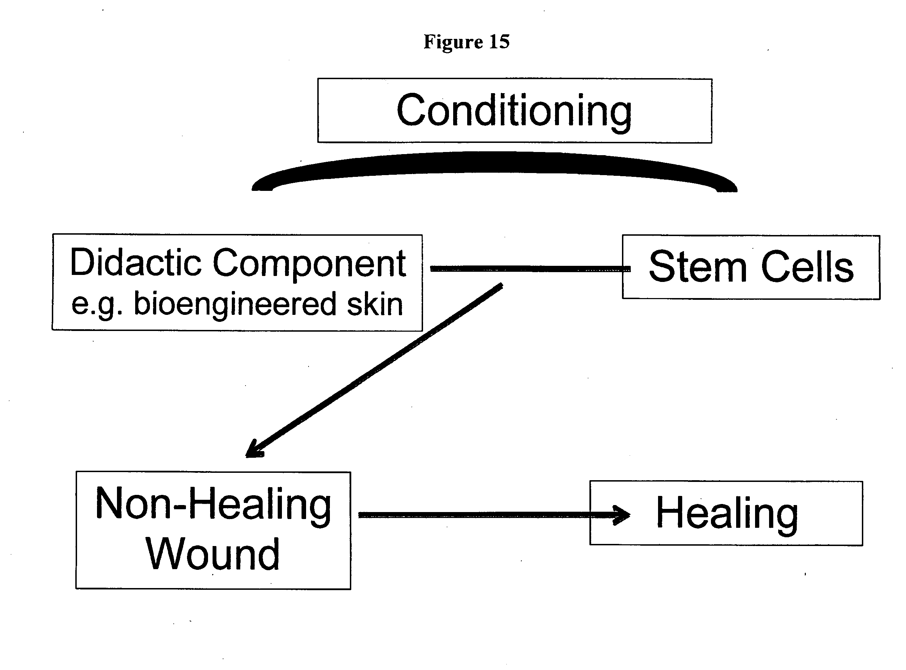

Figure 15. Provides a diagram of the process for conditioning the didacitic component and its application to the wound.

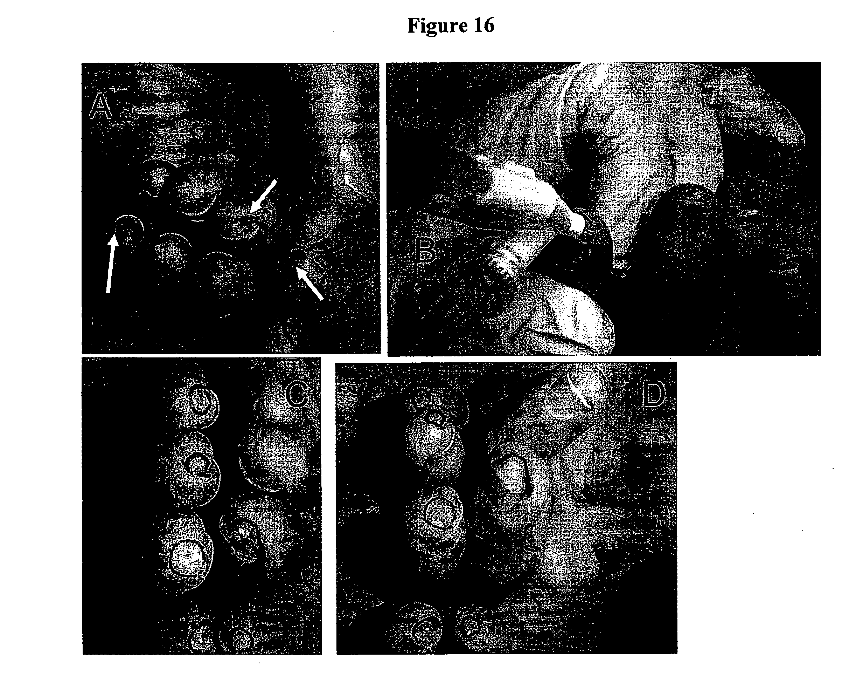

Figures 16 A - D. Illustrates scleroderma finger ulcers (ischemic) treated with MSC (dripped, not sprayed). (A) shows Ulcers and pitting scars at baseline; (B) shows application of MSC in fibrin; (C) shows ulcers and pitting scars outlined; and (D) shows the application of the fibrin/MSC sealant.

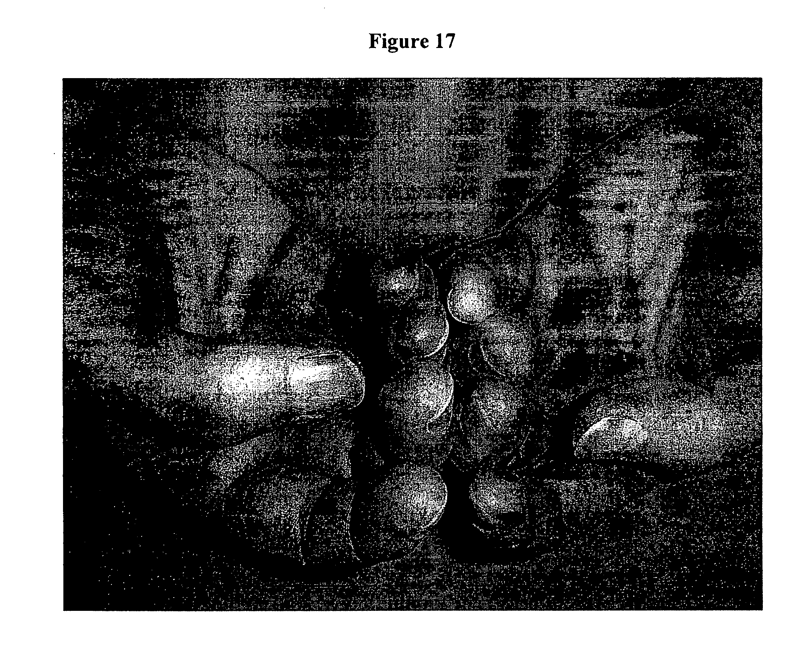

Figure 17. Shows the results of scleroderma finger ulcers (ischemic) treated with MSC, which remained healed after 8 weeks.

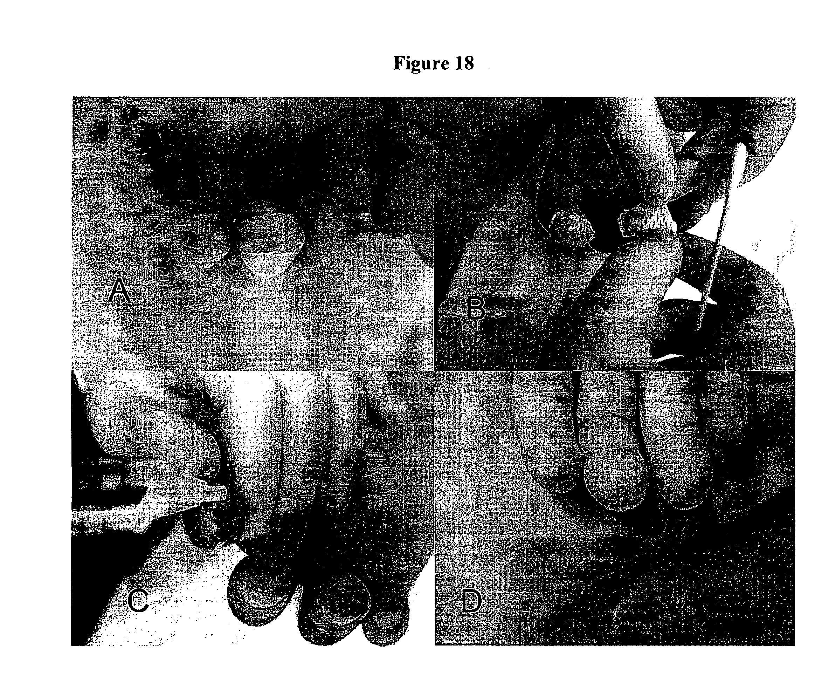

Figures 18 A - D. Illustrates scleroderma finger ulcers (ischemic) treated with MSC and covered with bioengineered skin (A) shows ulcers and pitting scars at baseline; (B) shows bioengineered skin over MSC gel; (C) shows MSC delivered in fibrin and the resultant gel polymer (there was immediate pain relief after treatment); (D) shows the result of healing after 4 weeks. The wounds stayed healed at 8 weeks.

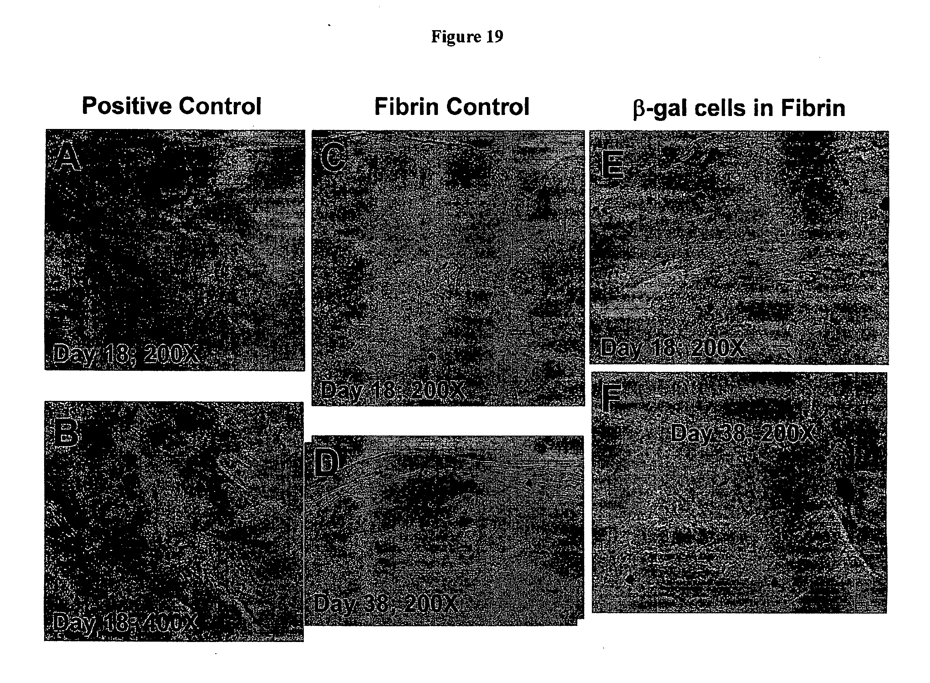

Figure 19 A-F. Compares β-gal staining in C57BL/6: application of MSC from β-galactosidase positive mice (A), (B) to syngeneic wounded mice in a C57BL/6 background (C), (D). Blue-stained cells show persistence of fibrin-delivered cells at 18 days(E) and 38 days (F).

### DETAILED DESCRIPTION

Fibrin sealants generally consist of two human plasma-derived components: (a) a highly concentrated Fibrinogen Complex (FC) composed primarily of fibrinogen and fibronectin along with catalytic amounts of Factor XIII and plasminogen and (b) a high potency thrombin. Fibrin sealants may also contain aprotinin. By the action of thrombin, (soluble) fibrinogen is at first converted into fibrin monomers which aggregate spontaneously and form a so-called fibrin clot. Simultaneously, factor XIII (FXIII) present in the solution is activated by thrombin in the presence of calcium ions to factor XIIIa. The aggregated fibrin monomers and any remaining fibronectin possibly present are cross-linked to form a high molecular weight polymer by new peptide bonds forming. By this cross-linking reaction, the strength of the clot formed is substantially increased. See *e*.*g*., U.S. Patent Publication No. 2008/0181879.

Generally, the clot adheres well to wound and tissue surfaces, which leads to the adhesive and haemostatic effect. Therefore, fibrin adhesives are frequently used as two-component adhesives which comprise a fibrinogen complex (FC) component together with a thrombin component which additionally contains calcium ions. One such commercially available fibrin sealant is TISSEEL (Baxter). TISSEEL consists of a two-component fibrin biomatrix that offers highly concentrated human fibrinogen to seal tissue and stop diffuse bleeding. Other commercially available fibrin sealants include BERIPLAST® (Behringwerke AG, Marburg/Lahn, FRG) and BIOCOL (CRTS, Lille, France).

The fibrin sealants of the present invention are produced by combining a fibrinogen complex (FC) component, a thrombin component, and further a cellular component. According to some embodiments, the cellular component may be added to the FC component before the addition of the thrombin component. According to some embodiments, the cellular component may be added to the thrombin component. According to some embodiments, cellular component may be added to the mixture of FC and thrombin before the components are allowed to form the fibrin gel.

The FC/thrombin ratio may be between the range of 2 to 10 mg/ml fibrinogen per 25 U/ml thrombin, which includes, for example, about 2 mg/ml fibrinogen per about 25 U/ml thrombin, about 2.5 mg/ml fibrinogen per about 25 U/ml thrombin, about 3 mg/ml fibrinogen per about 25 U/ml thrombin, about 3.5 mg/ml fibrinogen per about 25 U/ml thrombin, about 4 mg/ml fibrinogen per about 25 U/ml thrombin, about 4.5 mg/ml fibrinogen per about 25 U/ml thrombin, about 5 mg/ml fibrinogen per about 25 U/ml thrombin, about 5.5 mg/ml fibrinogen per about 25 U/ml thrombin, about 6 mg/ml fibrinogen per about 25 U/ml thrombin, about 6.5 mg/ml fibrinogen per about 25 U/ml thrombin, 7 about mg/ml fibrinogen per about 25 U/ml thrombin, about 7.5 mg/ml fibrinogen per about 25 U/ml thrombin, about 8 mg/ml fibrinogen per about 25 U/ml thrombin, about 8.5 mg/ml fibrinogen per about 25 U/ml thrombin, about 9 mg/ml fibrinogen per about 25 U/ml thrombin, about 9.5 mg/ml fibrinogen per about 25 U/ml thrombin, and about 10 mg/ml fibrinogen per about 25 U/ml thrombin. The FC/thrombin ratio is preferably between the range of about 5 mg/ml fibrinogen per about 25 U/ml thrombin. Preferred ranges for the FC/thrombin ratio include between about 4 to about 6 mg/ml fibrinogen per about 25 U/ml thrombin, between about 4.5 to about 6 mg/ml fibrinogen per about 25 U/ml thrombin, between about 5 to about 6 mg/ml fibrinogen per about 25 U/ml thrombin, between about 5.5 to about 6 mg/ml fibrinogen per about 25 U/ml thrombin, between about 4 to about 5.5 mg/ml fibrinogen per about 25 U/ml thrombin, between about 4 to about 5 mg/ml fibrinogen per about 25 U/ml thrombin, and between about 4 to about 4.5 mg/ml fibrinogen per about 25 U/ml thrombin.

According to preferred embodiment, the fibrinogen density/concentration is between the range of about 2 to about 10 mg/ml, which includes, for example, about 2 mg/ml, about 2.5 mg/ml, about 3 mg/ml, about 3.5 mg/ml, about 4 mg/ml, about 4.5 mg/ml, about 5 mg/ml, about 5.5 mg/ml, about 6 mg/ml, about 6.5 mg/ml, 7 about mg/ml, about 7.5 mg/ml, about 8 mg/ml, about 8.5 mg/ml, about 9 mg/ml, about 9.5 mg/ml, and about 10 mg/ml fibrinogen. The fibrinogen density is preferably between the range of about 2 to about 5 mg/ml. Preferred ranges for the fibrinogen density/concentration include between about 4 to about 6 mg/ml, between about 4.5 to about 6 mg/ml, between about 5 to about 6 mg/ml, between about 5.5 to about 6 mg/ml, between about 4 to about 5.5 mg/ml, between about 4 to about 5 mg/ml, between about 4 to about 4.5 mg/ml, between about 2 to about 6 mg/ml, between about 2 to about 5 mg/ml, between about 2.5 to about 5 mg/ml, between about 3 to about 5 mg/ml, and between about 3.5 to about 5 mg/ml. Thrombin is added to the fibrinogen in an amount sufficient to form a polymerized gel within between about 3 seconds to about 120 seconds. Preferably, between about 3 seconds to about 60 seconds, between about 3 seconds to about 30 seconds, between about 3 seconds to about 20 seconds, between about 3 seconds to about 10 seconds, between about 10 seconds to about 60 seconds, between about 20 seconds to about 60 seconds, between about 30 seconds to about 60 seconds, and between about 30 seconds to about 120 seconds

According to some embodiments, the foundation for the wound sealants of the present invention may be any protein other than fibrinogen/fibrin or any non-protein polymer (*e*.*g*., CAVILON™ from 3M) that is capable of forming a matrix and capable of supporting or encapsulating the cells or cellular component. The density/concentration of the polymer may be between 2.5 to 10 mg/ml. The polymer density/concentration may be between the range of about 2 to about 10 mg/ml, which includes, for example, about 2 mg/ml, about 2.5 mg/ml, about 3 mg/ml, about 3.5 mg/ml, about 4 mg/ml, about 4.5 mg/ml, about 5 mg/ml, about 5.5 mg/ml, about 6 mg/ml, about 6.5 mg/ml, 7 about mg/ml, about 7.5 mg/ml, about 8 mg/ml, about 8.5 mg/ml, about 9 mg/ml, about 9.5 mg/ml, and about 10 mg/ml. The polymer density is preferably between the range of about 2 to about 5 mg/ml. Preferred ranges for the polymer density include between about 4 to about 6 mg/ml, between about 4.5 to about 6 mg/ml, between about 5 to about 6 mg/ml, between about 5.5 to about 6 mg/ml, between about 4 to about 5.5 mg/ml, between about 4 to about 5 mg/ml, between about 4 to about 4.5 mg/ml, between about 2 to about 6 mg/ml, between about 2 to about 5 mg/ml, between about 2.5 to about 5 mg/ml, between about 3 to about 5 mg/ml, and between about 3.5 to about 5 mg/ml.

According to preferred embodiments, the fibrin sealants are in the form of a polymerized gel or gel spray. Concentration of each of the components should be optimized to prevent running after application.

According to preferred embodiments, the fibrin sealants comprise a therapeutic effective amount a stem/progenitor cells. Cells for the cellular component are preferably collected from an autologous, allogeneic, or heterologous human or animal source. An autologous animal or human source is more preferred. Stem/progenitor cell compositions are then prepared and isolated as described herein. For example, the fibrin sealants of the present invention comprise a cellular component comprising stem/progenitor cells at a concentration of about 10⁶ to 10²⁰ cells/ cm². According to some embodiment, the cellular component comprises a dosage of stem cells of at least about 1.5 - 2.0 X 10⁶ stem cells per cm² or greater (*e.g.,* greater than about 10⁷ cells/ cm², greater than about 10⁸ cells/ cm², greater than about 10⁹ cells/ cm², greater than about 10¹⁰ cells/ cm², greater than about 10¹¹ cells/ cm², greater than about 10¹² cells/ cm², greater than about 10¹³ cells/ cm², greater than about 10¹⁵ cells/ cm², or greater than about 10²⁰ cells/ cm²). Enriched stem cell preparations may also be used.

The compositions and methods of the invention disclosed herein are useful for treating a patient having acute or chronic wounds. Chronic wounds include, but are not limited to the following: chronic ischemic skin lesions; scleroderma ulcers; arterial ulcers; diabetic foot ulcers; pressure ulcers; venous ulcers; nonhealing lower extremity wounds; ulcers due to inflammatory conditions, and/or long-standing wounds. Although particular embodiments are exemplified herein, it is understood that a similar approach can also be used to treat other types of wounds using suitable autologous and/or allogeneic cells.

The compositions and methods of the invention disclosed herein are useful for treating a patient to ameliorate the formation of scars at a wound site. According to preferred embodiments, methods are provided to ameliorate, reduce, or decrease the formation of scars in a patient that has suffered a burn injury. According to preferred embodiments, methods are provided to treat, reduce the occurrence of, or reduce the probability of developing hypertrophic scars in a patient that has suffered an acute or chronic wound or injury.

The compositions and methods of the present invention may be used to treat chronic ischemic skin lesions. Accordingly to some embodiments, a composition comprising a suspension of stem/progenitor cells applied on the surface of and around chronic skin lesions, such as chronic ischemic skin lesions, chronic skin ulcers, and/or diabetic foot ulcers. Other types of chronic skin lesions include neurophatic and ischemic chronic cutaneous lesions (*e*.*g*., low-grade lesions, grade IV lesions, grade V lesions, *etc.*)*.*

The fibrin sealants of the present invention may be administered to a subject using techniques well-known in the art, for example by injection or spray at the desired site, endoscopically, using a sponge-like carrier, pre-formed sealant or other methods known in the art. In one embodiment, the sealant is injected or sprayed and allowed to form a gel *in situ.*

According to some embodiments, the fibrin sealants of the present invention may be delivered as a fine spray. According to preferred embodiments, the fibrin sealants of the present invention are applied to wounds using a fibrin polymer spray system with a double-barreled syringe. For this, the fibrinogen component may be combined with the cellular component and this combination is applied as a using a fibrin polymer spray system with a double-barreled syringe that is capable of simultaneously applying the combined FC/cellular components and the thrombin component. The fibrinogen and thrombin polymerize to fibrin immediately and on contact with the wound bed.

According to preferred embodiments, the fibrin sealant (*e.g*., polymerized fibrin gel) may be applied topically to the site of the wound. For example, the fibrin sealant may be applied to topically to the site of the wound as a gel and spread to evenly cover the surface area of the wound (*e.g*., non-healing wound). According to preferred embodiments, a didactic component, which refers to an organized structure (*e*.*g*., polymer or scaffold) that provides a niche, habitat, or structural support that enables the propagation and differentiation of the stem cells. According to some embodiments, the didactic component is a skin substitute or extracellular matrix (ECM) material. Skin substitutes include, but are not limited to, the following: acellular dressing (*e*.*g*., collagen bound to nylon fabric or mesh); autologous epidermal graft; acellular, allogeneic dermal graft; bovine collagen and chondroitin-6-sulfate; pig intestinal mucosa; human fibroblasts in an absorbable matrix; human fibroblasts and keratinocytes in a bovine collagen sponge; human fibroblasts and keratinocytes in a bovine collagen matrix. See *e*.*g*., U.S. Patent Publication No. 2007/0274963.

According to some embodiments, the fibrin polymer spray system uses a very low CO₂ flow (*i.e.,* less than about 10 p.s.i.) for fibrin delivery. Preferred ranges include from about 1 p.s.i. to about 10 p.s.i., from about 1 p.s.i. to about 9 p.s.i., from about 1 p.s.i. to about 8 p.s.i., from about 1 p.s.i. to about 7 p.s.i., from about 1 p.s.i. to about 6 p.s.i., from about 1 p.s.i. to about 5 p.s.i., from about 1 p.s.i. to about 4 p.s.i., from about 1 p.s.i. to about 3 p.s.i., from about 1 p.s.i. to about 2 p.s.i., from about 2 p.s.i. to about 5 p.s.i., from about 3 p.s.i. to about 5 p.s.i., or from about 4 p.s.i. to about 5 p.s.i.. According to preferred embodiments, the fibrin polymer spray system uses a CO₂ flow at less than about 5 p.s.i. for fibrin delivery. According to preferred embodiments, the fibrin polymer spray system uses a CO₂ flow at less than about 4.5 p.s.i. for fibrin delivery. According to preferred embodiments, the fibrin polymer spray system uses a CO₂ flow at less than about 4 p.s.i. for fibrin delivery. According to preferred embodiments, the fibrin polymer spray system uses a CO₂ flow at less than about 3.5 p.s.i. for fibrin delivery. According to preferred embodiments, the fibrin polymer spray system uses a CO₂ flow at less than about 3 p.s.i. for fibrin delivery. According to preferred embodiments, the fibrin polymer spray system uses a CO₂ flow at less than about 2.5 p.s.i. for fibrin delivery. According to preferred embodiments, the fibrin polymer spray system uses a CO₂ flow at less than about 2 p.s.i. for fibrin delivery.

Both fibrinogen (containing the stem/progenitor cells) and thrombin may be diluted to optimally deliver a polymerized gel that immediately adheres to the wound, without run-off, and yet allows the stem/progenitor cells to remain viable and migrate from the gel.

The fibrin sealant of the present invention may be administered to the site of the wound as needed during the course of the healing process, such as once, twice, three times, four times, five times, ten times, twenty times, or more. According to preferred embodiments, the fibrin sealants are administered to the patient for at least two applications at least 1 week apart. According to preferred embodiments, the fibrin sealants are administered to the patient for at least two applications at least 2 weeks apart. According to preferred embodiments, the fibrin sealants are administered to the patient for at least three applications at least 1 week apart. According to preferred embodiments, the fibrin sealants are administered to the patient for at least three applications at least 2 weeks apart. According to preferred embodiments, the fibrin sealants are administered to the patient for at least four applications at least 1 week apart. According to preferred embodiments, the fibrin sealants are administered to the patient for at least four applications at least 2 weeks apart. According to preferred embodiments, the fibrin sealants are administered to the patient for at least five applications at least 1 week apart. According to preferred embodiments, the fibrin sealants are administered to the patient for at least five applications at least 2 weeks apart.

According to some embodiments, the present invention provides topical compositions, and uses thereof, wherein the compositions contain stem/progenitor cells that are impregnated or seeded within a fibrin matrix. The stem/progenitor cells used in the composition and methods of the present invention may be allogenic or autologous stem/progenitor cells. Potential sources of stem cells include human embryonic stem (hES) cells, stem cells collected from umbilical cords, stem/progenitor cells collected from peripheral blood, bone marrow-derived cells, and adult stem cells, pluripotential stem cells, embryonic stem cells, very small embryonic-like stem cells, and cells that have been previously aliquoted and/or cyropreserved.

According to preferred embodiments, the cellular component of the fibrin sealants of the present invention may include adult autologous bone marrow-derived stem cells, adult autologous PBSCs, or adult autologous MSCs. According to preferred embodiments, the cellular component of the fibrin sealants of the present invention two or more of adult autologous bone marrow-derived stem cells, adult autologous PBSCs, or adult autologous MSCs in admixture.

An adult stem cell, or somatic stem cell, is an undifferentiated cell found among differentiated cells in a tissue or organ. An adult stem cell can renew itself, and can differentiate to yield the major specialized cell types of the tissue or organ. The primary role of adult stem cells in a living organism is to maintain and repair the tissue in which they are found.

The non-hematopoietic component of bone marrow includes multipotent mesenchymal stem cells (MSC) capable of differentiating into fat, bone, muscle, cartilage, and endothelium. According to preferred embodiments, MSCs are used in the methods and compositions of the present invention. Bone marrow derived stem cells are primarily found in the insides of long bones (legs, hips, sternum *etc.*) and comprise the "bone marrow". These stem cells may leave the bone marrow and circulate in the blood stream. The physical steps of collecting stem cells may comprise those steps known in the art.

Stem cells offer the possibility that some structures within the wound may be reconstituted. The bone marrow is an important source of hematopoietic stem cells that regularly regenerate components of the blood, and non-hematopoietic stem cells including mesenchymal stem cells (MSC). There are several potential mechanisms by which autologous stem cells could significantly contribute to wound healing. Under appropriate conditions stem cells can rejuvenate or rebuild tissue compartments. Falanga, Lancet 366(9498): 1736-43, 2005. Resident dermal fibroblasts in non-healing wounds have acquired an abnormal phenotype that is not conducive to appropriate tissue repair. Falanga et al., Tissue Eng. 2007 Jun; 3(6):1299-312. Specifically, fibroblasts cultured from non-healing wounds are unresponsive to the action of certain growth factors, such as platelet-derived growth factor-BB (PDGF-BB) and transforming growth factor-β1 (TGF-β1). The unresponsiveness to TGF-β1 may be due to down regulation of type II TGF-β receptors and decreased phosphorylation of key signaling molecules, including Smad3 and MAPK.

### Collection of stem cells

The stem/progenitor cells *(e.g.,* MSCs PBSCs, VSELs, *etc*.) of the present invention may be collected from bone marrow, peripheral blood (preferably mobilized peripheral blood), spleen, cord blood, and combinations thereof. The stem/progenitor cells may be collected from the respective sources using any means known in the art. Generally, the method of collecting stem/progenitor cells from a subject will include collecting a population of total nucleated cells and further enriching the population for stem/progenitor cells.

According to some embodiments, the stem/progenitor cells (*e.g.*, hematopoietic stem cells and/or MSCs) of the present invention are collected from bone marrow. Bone marrow derived cells may be collected using any methods known in the art. According to some embodiments, bone marrow aspirates may be obtained from patients. According to some embodiments, single bone marrow aspirates may be obtained from patients.

According to preferred embodiments, the stem cells/progenitor cells are very small embryonic-like (VSEL) stem cells. See *e.g*., WO/2007/067280, International Application No.: PCT/US2006/042780.

In some embodiments, the VSEL stem cells or derivatives thereof comprise CD34⁺/lin⁻/CD45⁻ or Sca-1⁺/lin⁻/CD45⁻very small embryonic-like (VSEL) stem cells. In some embodiments, the VSEL stem cells are about 3-4 µm in diameter, express at least one of SSEA-1 , Oct-4, Rev-1, and Nanog, posses large nuclei surrounded by a narrow rim of cytoplasm, and have open-type chromatin (euchromatin).

In some embodiments, the population of CD45⁻ cells comprising VSEL stem cells or derivatives thereof is isolated from a source in the human or the mouse selected from the group consisting of bone marrow, peripheral blood, spleen, cord blood, and combinations thereof. In some embodiments, the one or more growth factors that induce embryoid body-like sphere formation of the VSEL stem cells or derivatives thereof comprise epidermal growth factor (EGF), fibroblast growth factor-2, and combinations thereof. In some embodiments, the one or more factors are provided to the VSEL stem cells or derivatives thereof by co-culturing the VSEL stem cells or derivatives thereof with C2C12 cells. In some embodiments, the VSEL stem cells are CXCR4⁺ and/or AC133⁺. In some embodiments, the presently disclosed methods and compositions further comprise selecting those cells that are HLA-DR⁻, MHC class I⁻, CD90⁻, CD29⁻, CD105⁻, or combinations thereof. The presently disclosed subject matter also provides embryoid body-like spheres comprising a plurality of very small embryonic-like (VSEL) stem cells.

According to preferred embodiments, the stem cells/progenitor cells are collected from the peripheral blood of an individual. According to a preferred embodiment, the stem cells may be collected by an apheresis process, which typically utilizes an apheresis instrument. The apheresis instrument looks very much like a dialysis machine, but differs in that it is a centrifuge while a dialysis machine uses filtration technology. Stem cell collection can be accomplished in the privacy of the donors own home or in a collection center. Blood is drawn from one arm then enters the apheresis instrument where the stem cells are separated and collected. The rest of the whole blood is then returned to the donor. A registered nurse (RN) or other approved personnel places a needle into both arms of the subject in the same manner as a routine blood collection. The RN then operates the apheresis instrument that separates the blood elements (red cells, white cells, plasma) collecting the stem cells and returning the rest of the whole blood to the donor. The collection of stem cells requires approximately 2-4 hours during which the subject is at rest. Shortly after the apheresis collection, the bone marrow releases more stem cells into the blood stream to replace the harvested stem cells. The amount of stem cells collected is a very small fraction of a person's stem cells. In a healthy individual, the stem cells can rapidly multiply and replace the lost stem cells. Thus, the procedures of the invention does not deplete the body of stem cells. Many hundreds of thousands of apheresis collections take place each year for platelets, red cells, plasma and stem cells. It has been shown to be safe and effective technology.

According to some embodiments, stem cells and/or progenitor cells are collected by the process of apheresis from adult or pediatric peripheral blood, processed to optimize the quantity and quality of the collected stem cells, optionally cryogenically preserved and used for autologous therapeutic purposes when needed after they have been thawed. According to a preferred embodiment, there is provided a method for collecting autologous adult stem cells from a human subject. The process may involve collecting adult stem cells from peripheral blood human subject using an apheresis process; at the time of collection, earmarking the collected cells for use by the human subject; and preserving the collected cells to maintain the cellular integrity of the cells. The human subject may be an adult human or non-neonate child. Accordingly, the above processes may further include the collection of adult or non-neonate child peripheral blood stem cells where the cells are then aliquoted into defined dosage fractions before cryopreservation so that cells can be withdrawn from storage without the necessity of thawing all of the collected cells.

Collection may be performed on any person, including adult or a non-neonate child. Furthermore, collection may involve one or more collecting steps or collecting periods. For example, collection (*e.g.*, using an apheresis process) may be performed at least two times, at least three times, or at least 5 times on a person. During each collecting step, the number of total nucleated cells collected per kilogram weight of the person may be one million (1 x 10⁶) or more (*e.g.,* 1 x 10⁷, 1 x 10⁸, 1 x 10⁹ 1 x 10¹⁰, 1 x 10¹¹, 1 x 10¹², 1 x 10¹³, is 1 x10¹⁴, 1 x 10¹⁵). In preferred embodiments, the number of cells collected in a single collection session may be equal or greater than 1x10¹⁵ total nucleated cells, or at least on the order of 10¹⁴, or 10¹³, or 10¹², or 10¹¹, or 10¹⁰, or 10⁹, or 10⁸, or 10⁷, or 10⁶, or 10⁵ total nucleated cells, depending on the weight and age of the donor.

Depending on the situation and the quantity and quality of stem/progenitor cells to be collected from the donor, it may be preferable to collect the stem/progenitor cells from donors when they are at an "adult" or a "matured" age (the term "adult" as used herein refers to and includes adult and non-neonate, unless otherwise used in a particular context to take a different meaning) and/or at a certain minimum weight. For example, stem/progenitor cells are collected when the subject is within a range from 10 to 200 kg in accordance with one embodiment of the present invention, or any range within such range, such as 20 to 40 kg. In addition or in the alternative, it may be required that the subject be of a certain age, within a range from 2-80 years old *(e.g.,* 2-10, 10-15, 12-18, 16-20, 20-26, 26-30, 30-35, 30-40, 40-45, 40-50, 55-60, 60-65, 60-70, and 70-80 years old) in accordance with one embodiment of the present invention.

The collected stem cells and/or progenitor cells also may be expanded using an *ex-vivo* process. For example, it may be necessary to expand and propagate a population of stem cells, partially differentiate stem cells to achieve a population of tissue specific progenitor cells, or to differentiate stem cells or progenitor cells into fully functional cells. A variety of protocols have been developed for the enrichment of such populations. See *e*.*g*., U.S. Patent No. 5,486,359, U.S. Patent No. 5,753,506, and U.S. Patent No. 5,736,396.

Any known protocol for the expansion or differentiation of stems cells or progenitor cells may be employed. For example, strategies employed may include culturing stem cells or progenitor cells: with or without different cocktails of early and late growth factors; with or without tissue specific growth or differentiation factors; with or without serum; in stationary cultures, rapid medium exchanged cultures or under continuous perfusion (bioreactors); and with or without an established cell feeder layer. In order to achieve maximal *ex-vivo* expansion of stem cells the following general conditions should be fulfilled: (i) differentiation should be reversibly inhibited or delayed and (ii) self-renewal should be maximally prolonged. Similarly, following cell expansion, it is important to have methods to induce differentiation of the expanded cell population, so as to covert the expanded cell population to mature functional cells or tissue.

### Stem Cell Potentiating Agent

The amount of stem/progenitor cells circulating in the peripheral blood cell may be increased with the infusion of cell growth factors prior to collection, such as, for example, granulocyte colony stimulating factor (G-CSF). The infusion of growth factors is routinely given to bone marrow and peripheral blood donors and has not been associated with any long lasting untoward effects. Adverse side effects are not common but include the possibility of pain in the long bones, sternum, and pelvis, mild headache, mild nausea and a transient elevation in temperature. The growth factor is given 1-6 days before peripheral blood stem/progenitor cells are collected. 1-6 days after G-SCF is infused the peripheral blood stem/progenitor cells are sterilely collected by an apheresis instrument.

There is provided a method of mobilizing a significant number of peripheral blood stem/progenitor cells comprising the administration of a stem cell potentiating agent. The function of the stem cell potentiating agent is to increase the number or quality of the stem/progenitor cells that can be collected from the person. These agents include, but are not limited to, G-CSF, GM-CSF, dexamethazone, a CXCR4 receptors inhibitor, Interleukin-1 (IL-1), Interleukin-3 (IL-3), Interleukin-8 (IL-8), PIXY-321 (GM-CSF/IL-3 fusion protein), macrophage inflammatory protein, stem cell factor, thrombopoietin and growth related oncogene, as single agents or in combination. There is provided a method of mobilizing a significant number of peripheral blood stem/progenitor cells comprising the administration of G-CSF to a predisease subject.

The G-CSF is administered to a predisease subject over a 1 to 6 day course, which ends upon apheresis of the subjects peripheral blood. Preferably, the G-CSF is administered to a predisease subject at least twice over a 2 to 6 day period. For example, G-CSF may be administered on day 1 and day 3 or may be administered on day 1, day 3, and day 5 or, alternatively, day 1, day 2, and day 5. Most preferably, G-CSF is administered to a predisease subject twice for consecutive days over a 3 day course. Thus, G-CSF is administered to a predisease subject on day 1 and day 2 followed by apheresis on day 3.

Additionally, a low dose G-CSF is administered to a subject. Thus, a subject may receive a dose of G-CSF of about 1 µg/kg/day to 8 µg/kg/day. Preferably, G-CSF is administered to a subject at a dose of about 2 to about 7 µg/kg/day or equivalent thereof. More preferably, G-CSF is administered to a subject at a dose of about 4 to about 6 µg/kg/day or equivalent thereof. For subcutaneous injections, the dose of G-CSF may be from about 50 µg to about 800 µg, preferably from about 100 µg to about 600 µg, more preferably from about 250 µg to 500 µg, and most preferably from about 300 µg to about 500 µg.

Antagonist or inhibitors of CXCR4 receptors may be used as a stem cell potentiating agents. Examples of CXCR4 inhibitors that have been found to increase the amount of stem/progenitor cells in the peripheral blood include, but are not limited to, AMD3100, ALX40-4C, T22, T134, T140 and TAK-779. See also, U.S. Patent No. 7,169,750.

These stem cell potentiating agents may be administered to the person before the collecting step. For example, the potentiating agent may be administered at least one day, at least three days, or at least one week before the collecting step. Preferably, the CXCR4 inhibitors are administered to a predisease subject at least twice over a 2 to 6 day period. For example, the CXCR4 inhibitors may be administered on day 1 and day 3 or may be administered on day 1, day 3, and day 5 or, alternatively, day 1, day 2, and day 5. Most preferably, the CXCR4 inhibitors are administered to a predisease subject twice for consecutive days over a 3 day course. Thus, according to the preferred embodiment, the CXCR4 inhibitors are administered to a predisease subject on day 1 and day 2 followed by apheresis on day 3.

The formulation and route of administration chosen will be tailored to the individual subject, the nature of the condition to be treated in the subject, and generally, the judgment of the attending practitioner. Suitable dosage ranges for CXCR4 inhibitors vary according to these considerations, but in general, the compounds are administered in the range of about 0.1 µg/kg to 5 mg/kg of body weight; preferably the range is about 1 µg/kg to 300 µg/kg of body weight; more preferably about 10 µg/kg to 100 µg/kg of body weight. For a typical 70 kg human subject, thus, the dosage range is from about 0.7 µg to 350 mg; preferably about 700 µg to 21 mg; most preferably about 700 µg to 7 mg. Dosages may be higher when the compounds are administered orally or transdermally as compared to, for example, i.v. administration.

### Stem Cell Processing

In some embodiments of the invention, after collection, the stem/progenitor cells are processed according to methods known in the art (see, for example, Lasky, L. C. and Warkentin, P. I.; Marrow and Stem Cell Processing for Transplantation; American Association of Blood Banks (2002)). In an embodiment of the invention, processing may include the following steps: preparation of containers (*e.g.*, tubes) and labels, sampling and/or testing of the collected material, centrifugation, transfer of material from collection containers to storage containers, the addition of cryoprotectant, *etc.* In some embodiments, after processing, some of the processed stem/progenitor cells can be made available for further testing.

The cells also may be processed, preferably before the preservation step is conducted. Processing may involve, for example, enrichment or depletion of cells with certain cell surface markers. Any cell surface marker, including the cell surface markers listed anywhere in this specification may be used as a criteria for enrichment or depletion. Furthermore, processing may involve analyzing at least one characteristic of one cell in the one population of stem/progenitor cells or the at least one population of non-stem/progenitor cells. The characteristic may be a DNA or RNA sequence. For example, the genomic DNA or RNA may be partially or completely sequenced (determined). Alternatively, specific regions of the DNA or RNA of a cell may be sequenced. For example, nucleic acids from a cell or a cell population may be extracted. Specific regions of these nucleic acid may be amplified using amplification probes in an amplification process. The amplification process may be, for example PCR or LCR. After amplification, the amplimers (products of amplification) may be sequenced. Furthermore, the DNA and RNA may be analyzed using gene chips, using hybridization or other technologies.

Tissue typing of specific kinds may be used for sample identification or for the use of these stem/progenitor cells for possible allogeneic use. This type of information may include genotypic or phenotypic information. Phenotypic information may include any observable or measurable characteristic, either at a macroscopic or system level or microscopic, cellular or molecular level. Genotypic information may refer to a specific genetic composition of a specific individual organism, including one or more variations or mutations in the genetic composition of the individual's genome and the possible relationship of that genetic composition to disease. An example of this genotypic information is the genetic "fingerprint" and the Human Leukocyte Antigen (HLA) type of the donor. In some embodiments of the invention the stem/progenitor cells will be processed in such a way that defined dosages may be identified and aliquoted into appropriate containers.

In preferred embodiments, the number of cells in the stem/progenitor cell-enriched population may be equal or greater than 2x10⁸ total nucleated cells, or at least on the order of 10⁷, or 10⁶, or 10⁵, or 10⁴, depending on the weight and age of the donor. Aliquoting of these cells may be performed so that a quantity of cells sufficient for one dose will be stored in one cryocyte bag or tube. This process constitutes a unique process for "unitized storage" enabling individuals to withdraw quantities of cells for autologous use without the necessity of thawing the total volume of cells in storage (further details discussed below).

### Stem Cell Enrichment or Sorting

The enrichment procedure preferably includes sorting the cells by size and/or cellular markers. For example, stem cells comprise approximately 0.1-1.0% of the total nucleated cells as measured by the surrogate CD34+ cells. Thus, stem cells may be sorted by their expression of CD34.

In one aspect of the invention, the cells collected by the methods of the invention may be sorted into at least two subpopulations which may be cryopreserved separately or together *(e.g.,* in the same vial). The at least two subpopulations of cells may be two subpopulation of stem/progenitor cells. However, the at least two subpopulation of cells may be (1) a stem cell population or a population enriched for stem/progenitor cells and (2) a non stem cell population or a population depleted for stem/progenitor cells. Furthermore, it is also envisioned that the two subpopulations (*i.e.,* (1) and (2) above) may be cryopreserved together.

Stem cells may be sorted according to cell surface markers that are associated with stem cells. Since it is one embodiment of the invention to enrich for stem cells, useful markers for cell sorting need not be exclusively expressed in stem cells. A cell marker that is not exclusively expressed in stem cell will nevertheless have utility in enriching for stem cells. It should noted also that markers of differentiated cells are also useful in the methods of the invention because these markers may be used, for example, to selectively remove differentiated cells and thus enrich stem cells in the remaining cell population. Markers, cell surface or otherwise, which may be used in any of the processes of the invention include, at least, the following:

| **Marker** | **Cell Type** | **Significance** |
|---|---|---|
| | | **Blood Vessel** |
| Fetal liver kinase-1 (Flk1) | Endothelial | Cell-surface receptor protein that identifies endothelial cell progenitor; marker of cell-cell contacts |

| | | **Bone** |
|---|---|---|
| Bone-specific alkaline phosphatase (BAP) | Osteoblast | Enzyme expressed in osteoblast; activity indicates bone formation |

| | | **Bone Marrow and Blood** |
|---|---|---|
| Bone morphogenetic protein receptor (BMPR) | Mesenchymal stem and progenitor cells | Important for the differentiation of committed mesenchymal cell types from mesenchymal stem and progenitor cells; BMPR identifies early mesenchymal lineages (stem and progenitor cells) |
| CD34 | Hematopoietic stem cell (HSC), satellite, endothelial progenitor | Cell-surface protein on bone marrow cell, indicative of a HSC and endothelial progenitor; CD34 also identifies muscle satellite, a muscle stem cell |
| CD34⁺, Sca1⁺, Lin⁻ profile | Mesencyhmal stem cell (MSC) | Identifies MSCs, which can differentiate into adipocyte, osteocyte, chondrocyte, and myocyte |
| CD38 | Absent on HSC Present on WBC lineages | Cell-surface molecule that identifies WBC lineages. Selection of CD34+/CD38- cells allows for purification of HSC populations |
| c-Kit | HSC, MSC | Cell-surface receptor on BM cell types that identifies HSC and MSC; binding by fetal calf serum (FCS) enhances proliferation of ES cells, HSCs, MSCs, and hematopoietic progenitor cells |
| Colony-forming unit (CFU) | HSC, MSC | Progenitor CFU assay detects the ability of a single stem cell or progenitor cell to give rise to one or more cell lineages, such as red blood cell (RBC) and/or white blood cell (WBC) lineages |
| Fibroblast colony-forming unit (CFU-F) | Bone marrow fibroblast | An individual bone marrow cell that has given rise to a colony of multipotent fibroblastic cells; such identified cells are precursors of differentiated mesenchymal lineages |
| Hoechst dye | Absent on HSC | Fluorescent dye that binds DNA; HSC extrudes the dye and stains lightly compared with other cell types |
| KDR | Hematopoietic stem cell and progenitor cell. | VEGF-receptor 2. Present in hematopoietic stem cells and progenitor cells. |
| Leukocyte common antigen (CD45) | WBC | Cell-surface protein on WBC progenitor |
| Lineage surface antigen (Lin) | HSC, MSC Differentiated RBC and WBC lineages | 13 to 14 different cell-surface proteins that are markers of mature blood cell lineages; detection of Lin-negative cells assists in the purification of HSC and hematopoietic progenitor populations |
| Muc-18 (CD146) | Bone marrow fibroblasts, endothelial | Cell-surface protein (immunoglobulin superfamily) found on bone marrow fibroblasts, which may be important in hematopoiesis; a subpopulation of Muc-18+ cells are mesenchymal precursors |
| Stem cell antigen (Sca-1) | HSC, MSC | Cell-surface protein on bone marrow (BM) cell, indicative of HSC and MSC Bone Marrow and Blood cont. |
| Stro-1 antigen | Stromal (mesenchymal) precursor cells, hematopoietic cells | Cell-surface glycoprotein on subsets of bone marrow stromal (mesenchymal) cells; selection of Stro-1+ cells assists in isolating mesenchymal precursor cells, which are multipotent cells that give rise to adipocytes, osteocytes, smooth myocytes, fibroblasts, chondrocytes, and blood cells |
| Thy-1 | HSC, MSC | Cell-surface protein; negative or low detection is suggestive of HSC |
| CD14 | monocytes | Monocyte differentiation to dendritic cells. |
| Platelet Endothelial Cell Adhesion Molecule (PECAM-1 or CD31) | neutrophils, macrophages | Endothelial cell adhesion |
| CD73 | Lymphocyte | Lymphocyte maturation cell marker |

| | | **Fat** |
|---|---|---|
| Adipocyte lipid-binding protein (ALBP) | Adipocyte | Lipid-binding protein located specifically in adipocyte |
| Fatty acid transporter (FAT) | Adipocyte | Transport molecule located specifically in adipocyte |
| Adipocyte lipid-binding protein (ALBP) | Adipocyte | Lipid-binding protein located specifically in adipocyte |

| | | **Liver** |
|---|---|---|
| B-1 integrin | Hepatocyte | Cell-adhesion molecule important in cell-cell interactions; marker expressed during development of liver |

| | | **Nervous System** |
|---|---|---|
| CD133 | Neural stem cell, HSC | Cell-surface protein that identifies neural stem cells, which give rise to neurons and glial cells |
| Glial fibrillary acidic protein (GFAP) | Astrocyte | Protein specifically produced by astrocyte |
| 04 | Oligodendrocyte | Cell-surface marker on immature, developing oligodendrocyte |
| CD166 | Neural cell marker | Neural cell marker; activated T-cells |

| | | **Pancreas** |
|---|---|---|
| Cytokeratin 19 (CK19) | Pancreatic epithelium | CK19 identifies specific pancreatic epithelial cells that are progenitors for islet cells and ductal cells |
| Nestin | Pancreatic progenitor | Structural filament protein indicative of progenitor cell lines including pancreatic |

| | | **Pluripotent Stem Cells** |
|---|---|---|
| Alkaline phosphatase | Embryonic stem (ES) embryonal carcinoma (EC) | Elevated expression of this enzyme is associated with undifferentiated pluripotent stem cell (PSC) |
| Alpha-fetoprotein (AFP) | Endoderm | Protein expressed during development of primitive endoderm; reflects endodermal differentiation Pluripotent Stem Cells |
| Bone morphogenetic protein-4 | Mesoderm | Growth and differentiation factor expressed during early mesoderm formation and differentiation |
| Brachyury | Mesoderm | Transcription factor important in the earliest phases of mesoderm formation and differentiation; used as the earliest indicator of mesoderm formation |
| Cluster designation 30 (CD30) | ES, EC | Surface receptor molecule found specifically on PSC |
| Cripto (TDGF-1) | ES, cardiomyocyte | Gene for growth factor expressed by ES cells, primitive ectoderm, and developing cardiomyocyte |
| GATA-4 gene | Endoderm | Expression increases as ES differentiates into endoderm |
| GCTM-2 | ES, EC | Antibody to a specific extracellular-matrix molecule that is synthesized by undifferentiated PSCs |
| Genesis | ES, EC | Transcription factor uniquely expressed by ES cells either in or during the undifferentiated state of PSCs |
| Germ cell nuclear factor | ES, EC | Transcription factor expressed by PSCs |
| Hepatocyte nuclear factor-4 (HNF-4) | Endoderm | Transcription factor expressed early in endoderm formation |
| Nestin | Ectoderm, neural and pancreatic progenitor | Intermediate filaments within cells; characteristic of primitive neuroectoderm formation |
| Neuronal cell-adhesion molecule (N-CAM) | Ectoderm | Cell-surface molecule that promotes cell-cell interaction; indicates primitive neuroectoderm formation |
| Oct-4 | ES, EC | Transcription factor unique to PSCs; essential for establishment and maintenance of undifferentiated PSCs |
| Pax6 | Ectoderm | Transcription factor expressed as ES cell differentiates into neuroepithelium |
| Stage-specific embryonic antigen-3 (SSEA-3) | ES, EC | Glycoprotein specifically expressed in early embryonic development and by undifferentiated PSCs |
| Stage-specific embryonic antigen-4 (SSEA-4) | ES, EC | Glycoprotein specifically expressed in early embryonic development and by undifferentiated PSCs |
| Stem cell factor (SCF or c-Kit ligand) | ES, EC, HSC, MSC | Membrane protein that enhances proliferation of ES and EC cells, hematopoietic stem cell (HSCs), and mesenchymal stem cells (MSCs); binds the receptor c-Kit |
| Telomerase | ES, EC | An enzyme uniquely associated with immortal cell lines; useful for identifying undifferentiated PSCs |
| TRA-1-60 | ES, EC | Antibody to a specific extracellular matrix molecule is synthesized by undifferentiated PSCs |
| TRA-1-81 | ES, EC | Antibody to a specific extracellular matrix molecule normally synthesized by undifferentiated PSCs |
| Vimentin | Ectoderm, neural and pancreatic progenitor | Intermediate filaments within cells; characteristic of primitive neuroectoderm formation |

| | | **Skeletal Muscle/Cardiac/Smooth Muscle** |
|---|---|---|
| MyoD and Pax7 | Myoblast, myocyte | Transcription factors that direct differentiation of myoblasts into mature myocytes |
| Myogenin and MR4 | Skeletal myocyte | Secondary transcription factors required for differentiation of myoblasts from muscle stem cells |
| CD36 (FAT) | Cardiac cell marker | Integral membrane protein |

| | | **Progenitor Cell Marker** |
|---|---|---|
| CD29 | | Late antigen receptor involved in cell-cell adhesions |

The pattern of markers express by stem cells may also be used to sort and categorize stem cells with greater accuracy. Any means of characterizing, including the detection of markers or array of markers, may be used to characterized and/or identify the cells obtained through the embodiments disclosed herein. For example, certain cell types are known to express a certain pattern of markers, and the cells collected by the processes described herein may be sorted on the basis of these known patterns. The table that follows provides examples of the identifying pattern or array of markers that may be expressed by certain cell types.

| **Cell Type** | **Markers** |
|---|---|
| Hematopoietic stem cell | C34, CD45, CXCR4 |
| Endothelial Progenitors Cells | CD34, CD73, CD133, CXCR4, KDR, anti-M IgG |
| Mesenchymal Stem Cells | CD34, CD45, CD90, CD105, CD106, CD44 |
| Very Small Embryonic Like Cell. (VSEL) | CD34⁺/lin⁻/CD45⁻ or Sca-1⁺/lin⁻/CD45 |

### In vitro Propagation of Stem Cells

MSCs, PBSCs, or other stem cell types must be present in sufficient numbers for meaningful topical application. Proper characterization of the cultured cells is also important. This step requires appropriate collection and separation of the bone marrow and the use of tissue culture techniques that can yield cells with a stable phenotype. An even greater challenge is the actual application of stem cells to a wound. The cells need to be delivered in a preparation that remains in contact with the wound bed and keeps them viable in the often-hostile wound microenvironment. Ultimately, the cells have to enter the wound to effect a therapeutic response.

Thus, according to preferred embodiments, the number of stem/progenitor cells collected during the collection process is sufficient for the therapeutic application (*e.g*., wound healing). That is, the *in vitro* propagation of the collected is not necessary. In some instances, however, the *in vitro* propagation and/or manipulation of the stem/progenitor cells may be desired.

The propagation of the stem cells may be achieved using any known method. The stem cells of the present invention may be propagated on: 1) a tissue culture substrate in a stem cell medium that favors the maintenance of stem cells in a undifferentiated or dedifferentiated condition; 2) on fibroblast feeder layers that support cell growth and proliferation and inhibition of differentiation; or 3) a combination of both 1 and 2. In a preferred embodiment, the tissue culture substrate is coated with an adhesive or other compound or substance that enhances cell adhesion the substrate *(e.g.,* collagen, gelatin, or poly-lysine, *etc.*)*.* Collagen-coated plates are most preferred. Where fibroblast feeder cells are utilized, mouse or human fibroblasts are preferably used; alone or in combination. It is preferred that the feeder cells are treated to arrest their growth, which may be accomplished by irradiation or by treatment with chemicals such as mitomycin C that arrests their growth. Most preferably, the fibroblast feeder cells are treated with mitomycin C. In preferred embodiments, the fibroblast feeder layer has a density of approximately 25,000 human and 70,000 mouse cells per cm², or 75,000 to 100,000 mouse cells per cm². Preferably, the stem cells are cultured for a period of 4 to 24 days, and preferably for a period of 7 to 14 days. Preferably, the stem cells are grown on a fibroblast feeder layer, such as mitomycin treated MEF cells, for a period of about 4 to 14 days, and preferably from 7 to 10 days.

According to preferred embodiments, cells are grown *in vitro* under conditions favoring the propagation of MSC or cells with the following profile: CD29+, CD44+, CD105+, CD166+, CD34-, CD45-.

### Kits

Kits are also contemplated within the scope of the invention. A typical kit can comprise a fibrin sealant comprising an FC and a thrombin component. In one embodiment, the kit further comprises stem/progenitor cells or an admixture of stem/progenitor for incorporation into the fibrin sealant. In one aspect, each component may be included in its own separate storage container, vial or vessel. In a related aspect, the stem/progenitor may be in admixture with the FC component, and the thrombin component may be in a separate storage container. In a related aspect, the stem/progenitor may be in admixture with the thrombin component, and the FC component may be in a separate storage container. In a related embodiment, the storage container is a vial, a bottle, a bag, a reservoir, tube, blister, pouch, patch or the like. One or more of the constituents of the formulation may be lyophilized, freeze-dried, spray freeze-dried, or in any other reconstitutable form. Various reconstitution media can further be provided if desired.

The components of the kit may be in either frozen, liquid or lyophilized form. It is further contemplated that the kit contains suitable devices for administering the fibrin gel to a subject. In a further embodiment, the kit also contains instructions for preparing and administering the fibrin sealant.

According to some embodiments, the fibrin sealants of the present invention, and kits thereof, may further comprise growth factors known to be involved in the healing process. Such growth factors include, but are not limited to platelet-derived growth factor-BB (PDGF-BB), transforming growth factor-β1 (TGF-β1), and platelet derived growth factor (PDGF). The growth factors may be added in any concentration that provides an adequate delayed release formulation, within a range of 1 ng/ml to 1 mg/mL of the growth factors. Exemplary concentrations of growth factor in the fibrin sealant include, but are not limited to 1 ng/ml, 5 ng/ml, 10 ng/ml, 15 ng/ml, 20 ng/ml, 40 ng/ml, 50 ng ml, 100 ng/ml, 250 ng/ml, 500 ng/ml, 1 µg/ml, 5 µg/ml, 10 µg/ml, 25 µg/ml, 50 µg/ml, 100 µg/ml, 250 µg/ml, 500 µg/ml, 750 µ g/ml and 1 mg/ml. See *e*.*g*., U.S. Patent Publication Nos. 2009/0075881 and 2008/0181 879.

### Definitions

As used herein the terms "fibrin sealant," "fibrin gel," "fibrin adhesive," "fibrin clot" or "fibrin matrix" are used interchangeably and refer to a three-dimensional network comprising at least a fibrinogen complex (FC) component and a thrombin component, which can act as a scaffold for cell growth and release of a bioactive materials over time.

The term "calcic thrombin" as used herein includes thrombin in the presence of calcium.

As used herein, "stem cells" refer to cells that can give rise to one or more cell lineages. Included are progenitor cells, totipotent cells, pluripotent cells, embryonic cells or post natal and adult cells. Also included are tissue-specific cells, including, but not limited to, cells committed to a particular lineage capable of undergoing terminal differentiation, cells that derive from tissue resident cells, and circulating cells that have homed to specific tissues.

It should be understood that there is a distinction between a "hematopoietic stem cells" or "hematopoietic pluripotent stem cell" and a "stem cell collected from the hematopoietic system." A "hematopoietic stem cells" or "hematopoietic pluripotent stem cell" is a stem cell that by differentiation, and division, can repopulate the various lineages of the hematopoietic system. Hematopoietic stem cells (HSC) are stem cells and the early precursor cells which give rise to all the blood cell types that include both the myeloid (monocytes and macrophages, neutrophils, basophils, eosinophils, erythrocytes, megakaryocytes/platelets and some dendritic cells) and lymphoid lineages (T-cells, B-cells, NK-cells, some dendritic cells). A hematopoietic pluripotent stem cell does not have to be collected from the hematopoietic system. For example, hematopoietic stem cells may be collected from gut, spleen, kidney or ovaries - tissues that are not part of the hematopoietic system.

Conversely, a "stem cell collected from the hematopoietic system," such as the "peripheral blood stem cells" or "PBSC" collected by, for example, an apheresis process may be a stem cell for all tissue types in the body. That is, a stem cell collected by apheresis process may be any stem cell, such as a neural stem cell, an adipose tissue stem cell, a liver stem cell, a muscle stem cell, or a hematopoietic stem cell, *etc.* Thus, a stem cell collected by the method of this disclosure may give rise to any lineage of cells in a mammalian body, such as, for example, a neural stem cell, an adipose tissue stem cell, a liver stem cell, a muscle stem cell, or a hematopoietic stem cell *etc.*

The term "therapeutically effective amount" is used to denote treatments at dosages effective to achieve the therapeutic result sought. Furthermore, one of skill will appreciate that the therapeutically effective amount of the composition of the invention may be lowered or increased by fine tuning and/or by administering more than one composition of the invention (*e.g.,* by the concomitant administration of different populations of cells such as genetically modified cells or type of stem/progenitor cells), or by administering a composition of the invention with another compound to enhance the therapeutic effect (e.g., synergistically). The invention therefore provides a method to tailor the administration/treatment to the particular exigencies specific to a given mammal. As illustrated in the following examples, therapeutically effective amounts may be easily determined for example empirically by starting at relatively low amounts and by step-wise increments with concurrent evaluation of beneficial effect. The methods of the invention can thus be used, alone or in combination with other well known wound healing therapies, to treat a patient having acute or chronic wounds. One skilled in the art will readily understand advantageous uses of the invention, for example, by reducing healing time and outcome for a patient suffering from acute or chronic wounds.

Technical and scientific terms used herein have the meaning commonly understood by one of skill in the art to which the present invention pertains, unless otherwise defined.

The methods of the present invention are intended for use with any subject that may experience the benefits of the methods of the invention. Thus, in accordance with the invention, "subjects", "patients" as well as "individuals" (used interchangeably) include humans as well as non-human subjects, particularly domesticated animals.

As used herein, an "allogeneic cell" refers to a cell that is not derived from the individual to which the cell is to be administered, that is, has a different genetic constitution than the individual. An allogeneic cell is generally obtained from the same species as the individual to which the cell is to be administered. For example, the allogeneic cell can be a human cell, as disclosed herein, for administering to a human patient such as a cancer patient.

As used herein, a "genetically modified cell" refers to a cell that has been genetically modified to express an exogenous nucleic acid, for example, by transfection or transduction.

### EXAMPLES

### Example 1: Participation of Bone Marrow Derived Cells in Cutaneous Wound Healing

This example illustrates that bone marrow might be a valuable source of stem cells for the skin and possibly other organs. Wounding could be a stimulus for bone marrow derived stem cells to travel to organs and aid in the regeneration of damaged tissue. In this example, green fluorescent protein (GFP) labeled bone marrow transplanted into non-GFP mice was used in order to determine the participation of bone marrow stem cells in cutaneous wounds. The results indicate that there are a significant number of bone marrow cells that traffic through both wounded and non-wounded skin. Wounding stimulated the engraftment of bone marrow cells to the skin and induced bone marrow derived cells to incorporate into and differentiate into non-hematopoietic skin structures.

It has long been known that bone marrow derived cells play a critical role in wound healing as wounds enroll many mature inflammatory cells which provide cytokines that orchestrate the healing process. Many less well-characterized bone marrow derived cells, however, also migrate to wounds and their fate is not clear.

In the experiments disclosed herein, a non green florescent protein (GFP) expressing mouse was used. The non-GFP mouse had been previously transplanted with bone marrow from a GFP expressing transgenic mouse. Excisional wounds were made in these stably transplanted mice and the fate of the transplanted GFP expressing bone marrow cells was followed as these wounds healed. Many more bone marrow cells than predicted were noted to be present in wounded skin. Some bone marrow cells also were noted to give rise to mature structures, including sebaceous glands and dermal blood vessels. Thus, bone marrow likely represents a greater resource for cells important for wound healing than was previously expected.

*Transplant protocol:* Bone marrow was obtained from GFP expressing transgenic C57BL/6-TgN(ACTbEGFP)1Osb mice (The Jackson Laboratory, Bar Harbor, ME). The donor cells were washed and suspended in Hanks Balanced Saline Solution (HBSS). Recipient (non-GFP expressing) C57BL/6 mice were irradiated with 400 cGy total body radiation. Within 4 h of irradiation, the recipient mice were given 2.5x10⁷ donor cells by tail vein injection. Chimerism of the transplanted mice was determined (by tail vein bleeding) to be 60-70% by flow cytometry 4 weeks after administration of donor cells.

*Wounding protocol:* Excisional wounds, biopsies and harvesting of wounded tissue were performed by lifting the skin with forceps and removing a full thickness portion of skin (to the subcutaneous fat) using a curved scissors. Two excisional wounds were performed on the backs of transplanted mice at day 0. Control transplanted mice were not wounded. On day 2, one of the two wounds from the wounded animals was excised and a skin biopsy was obtained from a transplanted control mouse. On day 21, the second wound from wounded animals was harvested and a skin biopsy was obtained from a transplanted control mouse. Samples were divided and placed in 10% buffered formalin or 4% paraformaldehyde overnight. Formalin fixed tissues were paraffin embedded and sectioned on a Microme microtome. Paraformaldehyde fixed sections were snap-frozen and sectioned on a cryostat.

*Processing of the sections:* Paraformaldehyde fixed cryostat prepared sections were analyzed for the presence of GFP) by direct examination with fluorescence microscopy using a FITC filter. These sections were also counterstained with DAPI. Paraformaldehyde tissue was post-fixed in formaldehyde and paraffin embedded for immunohistochemistry staining with anti-GFP andtibody (Abcam, Cambridge, UK), and anti-pan-cytokeratin (Boehringer Mannheim Biochemicals, Roche Applied Science, Indianapolis, IN) was performed. Background was controlled with the use of a Vector M.O.M. peroxidase kit (Vector Labs, Burlingame, CA). *Mobilization:* Three months post GFP marrow cell infusion; mice were divided into three groups. One was a control group that was not further treated. The other two groups received two excisional wounds as described above. The wounded groups differed in that one group was given G-CSF twice daily for 4 consecutive days before wounding and on the day of wounding (total 5 days). The time of excisional wounding was counted as day 0 for all groups.

*Results:* Skin obtained from non-wounded, non-transplanted mice did not exhibit green fluorescence. Non-wounded transplanted mice contained scattered fluorescent cells within the dermis. Most of the cells appeared spindle shaped or dendritic and therefore could represent inflammatory cells such as tissue macrophages. There was a mild inflammatory infiltrate noted in (nonwounded) transplanted mice on routinely prepared hematoxylin & eosin (H & E) prepared sections, which was slightly higher in than in non-transplanted mice. Elements of the transplantation procedure, such as irradiation, could be in part responsible for this slight increase in inflammation. Ongoing engraftment of transplanted cells or a mild graft vs. host reaction are also possible. Histopathologic features of graft vs. host disease, however, were not observed in any of the specimens. The effect of radiation could also have been to locally reduce the number of resident progenitor cells. This may have created "room" or altered the competitive pressures for the bone marrow cells to repopulate the area.

In the wounded mice at day 2, there was a significant inflammatory infiltrate in both the G-CSF mobilized and non-mobilized groups. In the G-CSF treated group, the inflammatory infiltrate was generally greater than the non-G-CSF treated group. The amount of GFP present in the wound due to the infiltrate and ruptured inflammatory cells focally obliterated the wound field with fluorescence in many cases. Interpretation of these sections for engraftment of cells was difficult in both wounded groups due to the high level of signal present.

At day 21, the amount of inflammation in the wounded groups seen at day 2 was mostly resolved. Several GFP positive (GFP+) blood vessels were noted in the dermis of both (mobilized and non-mobilized) wounded groups. There were GFP+ cells noted in the hair follicle, striated muscle of the dermis, sebaceous glands and epidermis in both wounded groups. There seemed to be more GFP+ cells, however, in the epidermis, hair follicles and sebaceous glands in the G-CSF treated mice. Hair follicle, sebaceous gland and epidermal GFP+ cells were also demonstrated to double label for keratin and GFP antibodies. These findings strongly support the idea that bone marrow may supply needed stem and/or progenitor cells to wounded cutaneous tissues.

It is interesting to note in these experiments that many of the GFP+ cells noted in hair follicles appeared to occur in or near the hair bulge region, the reputed source of epidermal stem cells. It is may be that the bulge region is a stem cell niche where circulating (hematopoietic) stem cells are able to find a favorable microenvironment and home. In one illustrated follicle several GFP+ cells are noted. These GFP+ cells seem to have divided and begun migrating up the follicular unit, much like a keratinocyte progenitor of stem cell might.

Bone marrow has long been known to participate in wound healing by providing inflammatory cells which produce cytokines and orchestrate a cascade of events. Recent evidence of plasticity in bone marrow stem cells suggest that bone marrow might also serve as a resource to provide skin progenitor cells. In this study, we have illustrated engraftment of bone marrow cells to skin using a labeled bone marrow transplantation model. While most of the cells noted in the skin are likely to be inflammatory in nature, many were not. Skin structures, such as blood vessels and sebaceous glands, incorporated several labeled cells in their makeup. This finding could only be seen 21 days after wounding in these studies as there were too many (inflammatory) cells obliterating the field at the earlier examined time point of 2 days. It is likely that engraftment of stem or progenitor cells into the skin occurs early. A different model, possibly using lineage dependent promoters to control GFP expression, will be needed to address the question of early engraftment of bone marrow stem cells into the skin. It is, however, clear that once the inflammatory infiltrate has dissipated, labeled cells are retained in the dermis.

Finding labeled cells incorporated into mature skin structures was not a common event. In wounded animals, most blood vessels, hair follicles and associated adnexal structures did not contain labeled cells. These animals did not exhibit any advantage or deficit in wound healing over control mice. Wounds healed at similar rates and histologically appeared comparable to controls. It is possible then that in an animal with good resident stem cells, significant recruitment from an outside source for stem cells would not be required. This could have lead to the limited amount of engraftment observed in skin structures. Animals with poor resident skin stem cells and impaired wound healing, similar to the situation seen in venous ulcers, might then exhibit greater engraftment. However, it should be noted that keratinocytes in this GFP transgenic express GFP poorly, so these observations may represent an underestimation of the extent of transdifferentiation.

The presence of labeled cells within mature skin structures does, however, provide evidence that engraftment of bone marrow derived cells is a functional event. These cells do not appear to simply reside in the skin but appear, even if to a limited extent, to become a component of several skin structures with morphologic features identical to the resident skin structures.

Attempts were made to deliver bone marrow derived skin progenitor and skin stem cells to wounded tissues. The cultured cells were placed as a suspension under an occlusive film applied over the wound. The results, however, were unsatisfactory in that the cell suspension could easily run off the wound and definite and reliable delivery of the cultured cells could not be insured. It is also noted that characterization of the cultured cells was not performed.

### Examples 2 - 12

The following examples describe the cell culture and characterization, delivery system, and successful use of topically applied autologous MSC to accelerate the healing of human and experimental murine wounds. A single bone marrow aspirate of 35-50mL was obtained from patients with acute wounds (n = 5) from skin cancer surgery and from patients with chronic, long-standing, nonhealing lower extremity wounds (n = 8). Cells were grown *in vitro* under conditions favoring the propagation of MSC, and flow cytometry and immunostaining showed a profile (CD29+, CD44+, CD105+, CD166+, CD34-, CD45-) highly consistent with published reports of human MSC. Functional induction studies confirmed that the MSC could differentiate into bone, cartilage, and adipose tissue. The cultured autologous MSC were applied up to four times to the wounds using a fibrin polymer spray system with a double-barreled syringe. Both fibrinogen (containing the MSC) and thrombin were diluted to optimally deliver a polymerized gel that immediately adhered to the wound, without run-off, and yet allowing the MSC to remain viable and migrate from the gel. Sequential adjacent sections from biopsy specimens of the wound bed after MSC application showed elongated spindle cells, similar to their *in vitro* counterparts, which immunostained for MSC markers.

Generation of new elastic fibers was evident by both special stains and antibodies to human elastin. The application of cultured cells was safe, without treatment-related adverse events. A strong direct correlation was found between the number of cells applied (greater than 1x10⁶ cells per cm² of wound area) and the subsequent decrease in chronic wound size (p = 0.0058). Topical application of autologous MSC also stimulated closure of fullthickness wounds in diabetic mice (db/db). Tracking of green fluorescent protein (GFP)+ MSC in mouse wounds showed GFP+ blood vessels, suggesting that the applied cells may persist as well as act to stimulate the wound repair process. These findings indicate that autologous bone marrow-derived MSC can be safely and effectively delivered to wounds using a fibrin spray system.

The following examples disclose and show the successful culture and propagation of MSC from human and mouse bone marrow for topical delivery to autologous animal and human wounds. The establishment of these cell cultures was rapid, and their characterized MSC phenotype was evident and stable in culture, as shown by morphology, flow cytometry, immunostaining, and functional assays. The experiments show that these cells can be successfully applied to the wound bed using a fibrin spray system. For this, concentrations of both fibrinogen and thrombin were modified so as to deliver a fine spray that polymerized to fibrin immediately and on contact with the wound bed. Using a very low CO₂ flow for fibrin delivery, there was no run-off of the preparation from the wound bed. MSC in the fibrin spray remained viable and were able to migrate from the fibrin matrix, as determined by both *in vitro* and *in vivo* studies. Thus, the application of autologous cultured MSC to human acute and chronic wounds is safely accomplished.

The applied cells appear to establish themselves in the wound bed. A decrease in size of the chronic wounds correlated very strongly and with a great degree of statistical significance with the number of MSC applied per cm² of the wound surface. Indeed, a concentration of 1x10⁶ cells per cm² was clearly required to stimulate a decrease in wound size. Animal studies showed that mouse autologous bone marrow-derived MSC can accelerate the healing of full-thickness wounds in db/db mice as well as their control littermates. Tracking of GFP+ MSC in full-thickness mouse wounds suggests that most of these cultured cells may not persist, in spite of the stimulation of wound healing. Taken together, these results indicate the feasibility of using a modified fibrin spray system to deliver cells to wounds, and also offer considerable promise that bone marrow-derived cultured MSC can accelerate healing.

Of crucial importance is that our studies were performed not only in experimental animal wounds but also in difficult-to-heal human wounds.

The autologous bone marrow- derived cultured stem cells were fully characterized to show their mesenchymal phenotype. Delivery of these cells in a fibrin spray of the present invention was useful for enhancing the healing of wounds. Fibrin is a well-tested system already in use to stop bleeding during surgery. Crosslinked fibrin stimulates cell attachment and spreading. Fibrin may be safe for cells, including MSC, and for the healing process.

The fibrinogen and/or thrombin concentrations were modified for the delivery of cells to a wound. Ideal concentrations of these components for developing fibrin constructs that, at least *in vitro*, allow fibroblasts to migrate onto tissue culture plastic. It was also determined that the readily available commercial fibrin preparation, which is used to control bleeding, could not be used in the same way to deliver cells. In fact, *in vivo* studies found that full concentrations of fibrinogen and thrombin encased the stem cells to the point that they appeared to be nonviable (not shown). The concentrations of fibrinogen and thrombin as described herein, however, appear to be effective in cell delivery, as indicated by our histological and immunofluorescence analysis and by the acceleration of wound healing.

In culturing the FICOLL®-separated bone marrow aspirate, we chose to test for a subset of high-yield cluster designation markers to provide information on the lineage of the cultured cells. While the cells were plastic adherent and had similar morphology to stromal cells, we were interested in confirming that the cells were indeed mesenchymal in origin. There is no specific MSC marker, and we therefore selected a group of markers. We chose CD29, CD44, CD90, CD105, and CD166 as they are commonly cited as MSC markers in the literature. See *e.g.,* Kassis et al., Bone Marrow Transplant 37(10): 967-76, 2006; Tuli et al., Stem Cells 21(6): 681-93, 2003.

For the sake of practicality and for developing a method that could be repeated on cultures from multiple patients, we chose this previously reported subset. Other markers described in reports of human MSC include STRO-1, CD73, and CD49e. Pediatric Research 63:5, 502-512. We also tested for CD34 and CD45 to insure that we were culturing the stromal component of the bone marrow, and not hematopoietic cells. Our cells were CD34 and CD45 negative, indicating that they are not hematopoietic cells or leukocyte precursors. Some investigators have proposed that early or primitive stromal elements should be CD34+ and thus exhibit more "stemness." Pediatric Research 63:5, 502-512. However, these experiments do show that the cultured cells could differentiate into bone, adipose, and cartilage components. Moreover, our goal was to develop an easily reproducible culture system that would insure that we were dealing with MSC and that MSC could be properly delivered topically and accelerate healing.

These experiments show the feasibility of fibrin as a delivery system in both acute and chronic human wounds. The results were the enhancement of the healing process and, equally important, no adverse effects were noted.

For chronic wounds, subjects were chosen with very difficult-to-heal wounds, which were present for a long time and were unresponsive to even advanced therapies. Thus, the results in these subjects are extremely promising. There was a strong and statistically significant correlation between the number of applied MSC and acceleration of healing. The optimal number of applied cells needs to be at least 1x10⁶ cells per cm² of the wound surface. This is very important information that could only be discovered empirically and by actually studying these difficult wounds. Identifying the number of cells is also critical for larger studies in the future. The results obtained with the acceleration of healing of full thickness wounds in db/db mice provide additional evidence for the effectiveness of MSC in stimulating wound repair. As expected, control littermates healed faster than db/db mice, although they too showed a statistically significant healing response to autologous mouse MSC.

Sequential adjacent sections were immunostained for different markers in order to properly and safely mark cells and track them from the fibrin gel into the wound. The results in both acute and chronic wounds showed that the introduced cells, positive for CD29 and for prolyl hydroxylase as a specific human fibroblast marker, can be found in the dermis, immediately under the site of fibrin gel delivery. The highly spindled morphological appearance was found in histological and immunostained sections, remarkably similar to what was documented in tissue culture, is also supportive of the fact that the stem cells did indeed migrate from the applied fibrin gel and mobilize into the dermis. Moreover, using two separate methods to detect elastic fibers, there was strong evidence that new elastic fibers may have been deposited in the dermis of full thickness in wounds treated with MSC. This possible regeneration of elastic fibers, which normally does not occur in healing wounds or in scars, is quite interesting. Similar accumulation of spindle cells having these markers in the upper dermis or new elastic fiber formation were not found in control sections where the wound received fibrin alone.

In order to further track the MSC applied to wounds, we performed experiments in mice by using GFP+ autologous MSC isolated from syngeneic strains. For imaging the immunofluorescence, we used a stringent system of optical filters to exclude the possibility of auto-fluorescence and other situations of false positive green fluorescence. Our results indicate that, at least at later time points, labeled MSC could not be found in clusters, except for very occasional individual cells. One explanation for this result is that cultured and topically applied MSC may participate in the stimulation of wound healing by the production of cytokines and/or stimulation of endogenous resident cells. Another explanation is that, because GFP is quite immunogenic, cells engineered with this protein are removed by the host immune system. We rarely observed GFP+ dermal blood vessels in the healed wound. At this point, we do not know whether this could represent cell fusion or a rare event of MSC conversion to endothelium.

In summary, these experiments describe methods for reliably culturing human and mouse MSC from bone marrow and for the delivery of these cells to human wounds and experimental murine wounds. The approach appears reliable and safe and is very promising in terms of effectively stimulating the repair process in injured tissue.

### Example 2: Vulture of bone marrow-derived cells

All clinical materials and components used in this study were approved by the Institutional Review Board (IRB) of Roger Williams Medical Center. To determine the validity of the topical delivery method and the safety of the approach, two types of human wounds were studied: acute wounds resulting from the removal of a non-melanoma skin cancer from the trunk or limbs and considered likely to heal properly with secondary intention but not ideally suitable for primary closure; and chronic wounds of the lower extremities and feet, which had not been healing for a period of at least 12 months in spite of appropriate standard care and more innovative approaches including topical application of PDGF and bioengineered skin products.

A single bone marrow aspirate (35-50mL) was taken from each patient's iliac crest. To prevent clotting, the aspiration needle was primed with sterile heparin sulfate (1000U/mL).

The bone marrow aspirate was aliquoted immediately after collection. Each 4mL of the aspirate was layered onto 3mL of sterile FICOLL-PAQUE™ Plus (GE Healthcare Biosciences, Piscataway, NJ) in 15mL conical tissue culture polypropylene tubes (Becton Dickinson Labware, Franklin Lakes, NJ). The resulting suspension was centrifuged at 400 g for 30 minutes at room temperature. The mononuclear layer at the interface between the FICOLL-PAQUE™ and plasma was removed and plated onto T-25 tissue culture flasks containing 7mL of media, consisting of basic MSC media supplemented with 10% mesenchymal stimulatory supplements (StemCell Technologies, Vancouver, BC, Canada). The flasks were incubated and kept at 378C, 5% carbon dioxide (CO2) for the initial 48 hours. The media were then removed and 7mL of fresh media were added every 3-4 days. When the cell monolayers achieved confluence (generally 1 week later), they were passaged to T-75 flasks and supplemented with 15mL of new media every 3-4 days. Periodically, cell cultures were tested for the presence of mycoplasma using the Mycoalert mycoplasma detection assay (Cambrex Bio Science, Baltimore, MD), and with appropriate positive and negative controls. The measurements were made by performing a bioluminescent assay.

### Example 3: Flow cytometry to characterized cultured cells

Flow cytometry analysis was used to determine specific markers on the cultured cells, from both mouse and human bone marrow aspirates and preparations. Cells in suspension were mixed with fluorochrome-conjugated antibodies against CD29, CD31, CD34, CD44, CD45, CD105, CD166, SCA-1 (BD Biosciences, St. Jose, CA). Appropriate isotype antibodies were used to control for nonspecific staining. Staining was performed according to manufacturer's recommendations. Immunostained cells were acquired and analyzed on a FACS Calibur flow cytometer (BD Biosciences), using cellQUEST software (BD Biosciences). Cells were first visualized on forward scatter versus side scatter, and a gate was constructed around viable cells, thus eliminating nonviable cells and debris. To insure the stability of the phenotype of cultured cells, flow cytometry was performed at passages 3-5 and again at passages 10-11.

On the day of cultured cell application, cell monolayers in T-75 flasks were trypsinized with 0.05% trypsin-EDTA (Invitrogen, Carlsbad, CA), collected in regular mesenchymal media, and centrifuged for 5 minutes, at 400 g at room temperature. The cells were then resuspended and washed twice with sterile saline, repelleting at the same centrifuge conditions. A sample of cell suspension was taken prior to the final spin for determining cell count using a hemacytometer and for calculating the number of cells delivered per cm² of the wound surface.

### Example 4: Immunohistochemistry for further characterization of cultured cells

Immunohistochemistry was performed as previously described by Butmarc et al. (Wound Repair Regen 12(4): 439-43, 2004), which is incorporated herein by reference in its entirety. Cells from passages 5 to 7 were trypsinized with 0.05% trypsin-EDTA (Gibco, Grand Island, NY), diluted in MSC media, and allowed to adhere to sterile glass slides for 15 minutes. These slides were then placed in 100x15mm dishes, covered with 10mL of MSC media, and allowed to grow at 37°C, 5% CO₂ for 24 hours. The slides were washed in phosphate buffered saline (PBS), air-dried, fixed in cold acetone, placed in 1xPBS, and immunostained. Cluster designation marker primary antibodies included the following: monoclonal CD29 (Serotec, Raleigh, NC),monoclonal CD34 (Immunotech, Marseilles, FR), monoclonal CD44, monoclonal CD45, monoclonal CD105, polyclonal CD117 (Dako Cytomation, Carpinteria, CA), and monoclonal CD90 (Oncogene, San Diego, CA). In addition, adjacent 4 mm tissue sections from formalin-fixed biopsy specimens of the wound bed were also analyzed with identical antibodies for MSC markers, as well as using a monoclonal antibody to human prolyl-4- hydroxylase beta (Acris antibodies, Germany, distributed by Novus Biologicals, Littleton, CO). To analyze whether tissue regeneration might be occurring, we focused on the reestablishment of elastic fibers. This was done using special stains (Verhoeff-vanGieson) aswell as a specific elastinmonoclonal antibody obtained from Vision Biosystems/Novocastra (Norwell, MA).

### Example 5: Functional assays to determine the differentiation capacity of cultured MSC

Bone marrow-derived cultured cells between passages 3 and 9 were tested for their ability to differentiate into osteocytes, chondrocytes, and adipocytes using specific differentiation assays (Cambrex). For positive controls an MSC strain (Cambrex POETICS™ human mesenchymal stem cells) was utilized. Negative controls consisted of the patients' cells grown in standard MSC media, and were used to demonstrate that the phenotypic changes were nonspontaneous. Briefly, for adipocyte differentiation, cells were plated onto 6-well plates and grown to confluence. They were then exposed to 3 cycles of adipogenic induction media alternating with adipogenic maintenance media. Negative control cells received adipogenic maintenance media alone. The cells were observed for oil droplet formation and were also stained with Oil Red O. This technique allowed bright orange-red staining of fat and blue staining of nuclei. To better emphasize cell outlines and nuclei, the cells were incubated with 1 mL of hematoxylin counterstain for 2.5 minutes. Pictures were taken using a Nikon Coolpix camera on an Olympus phase-contrast inverted scope. Osteogenic induction requires cells aliquoted into 6-well plates to be grown to confluence in osteogenic induction media. To measure calcium deposition as a marker of bone formation, cells were washed, scraped off the plates, and digested overnight with hydrochloric acid. A calcium reporter assay (Stanbio Total Calcium LIQUICOLOR®) was then used to determine the amount of calcium in the cell lysate. Per this procedure, 550nm absorbance ratings were made using a spectrophotometer. Cells being induced into chondrocytes were counted and spun into pellets containing 3x10⁴ to 4x10⁵ cells. The pellets were supplemented with chondrogenic induction media containing TGF-β3 every 2-3days for 4 weeks and kept under standard conditions. The pellets were then processed, externally stained with eosin, embedded, and cut into 4-mm sections for staining with Safranin O. Known cartilage tissue was used as a positive staining control and cell pellets that had been kept in regular mesenchymal media without induction served as negative controls.

### Example 6: Fibrin spray system to delivery cells topically to wounds

The fibrin delivery method made use of the TISSEEL VH® fibrin sealant system and the TISSOMAT® application device and spray set (Baxter Healthcare, Glendale, CA). This preparation contains human fibrinogen and thrombin. The following protocol was used to make a fibrin gel with final concentrations of 5 mg/mL fibrinogen and 25 U/mL thrombin, utilizing a 1mL TISSEEL VH® kit (Baxter). This kit utilizes two liquid phases that can be either extruded through a dual chamber applicator or sprayed through the applicator with an inert gas carrier. To make the thrombin component, 1mL of calcium chloride (CaCl₂) mixture from the kit was added using a sterile syringe to the 1mL bottle of thrombin, and the mixture was allowed to dissolve. One part of this solution was then added to 9 parts of sterile 30mM CaCl₂ in normal saline (0.9% sodium chloride [NaCl]). The fibrinogen/sealer protein component was made by adding 1mL of sterile normal saline to the sealer protein bottle. One part of this solution was then added to 9 parts of sterile normal saline. The protease inhibitor aprotinin included with the kit was not used. All solutions were used within 4 hours. The total volume of fibrin gel was predetermined by the size of the wound to be covered. At the time of application to each wound, a small amount of the fibrin gel was placed on a tissue culture plate, covered in media, and incubated under standard conditions to verify and confirm cell viability and migration of the cells from the fibrin.

### Example 7: Mouse experiments to determine cell delivery and efficacy with the fibrin spray system

For initial *in vivo* screening of cell delivery using the fibrin spray, red fluorescence-labeled fluorescent protein (FP)+ mouse MSC were delivered to wounds created in the back of C57BL/6 mice. For red-fluorescence labeling, cells were stained and washed using the PKH26 red fluorescent linker kit (Sigma, St. Louis, MO) 2 hours prior to application. Just prior to wounding, the backs of mice were shaved and anesthesia was administered. A 1x1.5 cm elliptical full thickness wound was made in the upper back of the mice. A polymer film (CAVILON™, 3M, St. Paul, MN) was used to cover the wound, followed by silicone dressings (Mepitel, Molnilcke, Sweden). The dressing was secured to the unwounded dorsal skin with clips. On day 5, the mice were sacrificed by CO₂ inhalation. The dressing was removed and the wounded area was excised. Frozen sections were prepared by snap freezing the skin in isopentane on OTC mounting compound. Thereafter, 5 µm sections were cut and observed for red fluorescence. In other experiments, mouse MSC cultures were established from the bone marrow of green fluorescent protein (GFP)+ mice (UBI-GFP/BL/6; Jackson Laboratory, Bar Harbor, ME) and db/db mice (BKS.Cg-m+/ +Leprdb/J, former name C57BLKS-m Lepr^{db}, Jackson Laboratory).

For actual wounding experiments, female mice (25-30 grams) were used under conditions previously described in Falanga et al. (Wound Repair Regen 12(3): 320-26, 2004), incorporated herein by reference in its entirety. The fibrin spray system was then used to spray the GFP+ cells onto the wound. In experiments designed to measure efficacy and tracking, we delivered GFP+ cultured MSC to full-thickness tail wounds of db/db mice and control (db/+) littermates using a model previously described in Falanga et al. (Wound Repair Regen 12(3): 320-26, 2004), incorporated herein by reference in its entirety. Imaging of histological and immunofluorescent sections for these experiments was performed using the Zeiss Axioplan 2 system with the Axio CAM, and sections were analyzed by filters for fluorescein isothiocyanate (FITC) (510-560 nm), light microscopy, Texas Red (645/75 nm), and DAPI (435-475 nm). Using these filters, a composite photomicrograph was obtained, which helped to determine the specificity of the green fluorescence.

### Example 8: Statistical analysis

The work on human subjects was analyzed by determining the effect of each application of MSC and any correlation with the cell numbers on subsequent change in wound size within the immediate 2-4 weeks following the topical application. This analysis was done using the nonparametric Spearman Rank Correlation with no Gaussian assumption. For further analysis of the number of cells needed for a clinically meaningful biological effect, the two-sided Fisher's Exact Test was used, which helped to determine the effect of greater or less than 1x10⁶ cells/cm2 of the wound on any decrease (at least 10%) in ulcer size. Comparisons with mice experiments were obtained using the Bonferroni Multiple Comparisons Test.

### Example 9: Cell culture and characterization, and delivery in a fibrin spray

No adverse events occurred during the harvesting of bone marrow from patients, and separation of the bone marrow aspirate using FICOLL® and subsequent cell culture also proceeded without problems. All cell cultures tested negative for mycoplasma. We first focused on the morphology of the cultured cells. This is shown in Figures 1A-D. After primary plating of the FICOLL®-separated bone marrow aspirate and change of media at 48 hours, fusiform or polygonal cells with multiple small, knob-like cytoplasmic projections could be seen adhering to the tissue culture plastic (Figure 1A). By approximately 5 days, many of the flasks contained spindle-shaped cells. In many cases, the cells were extremely elongated, and in fact appeared to be aligning themselves end to end along their long axis (Figure 1B). Cells for application to patients were used within the first 10 passages. After multiple passages (>10-12) the cell morphology became more bizarre and polygonal (Figure 1C). Occasionally and in very early passages (<5), one in approximately 10 culture dishes, more complex structures formed, consisting of many cells adhering to one another along their sides and their long axis, appearing like a three dimensional structure (Figure 1D). In general, within a week the cells became confluent. Cell proliferation slowed with increasing *in vitro* passage, so that doubling times, initially 3-4 days, increased to 14 days or more with passages greater than 10-12. When placed in tissue culture flasks within the fibrin polymer matrix as described above, the cells were able to migrate out of the fibrin onto tissue culture plastic as early as 4 hours (Figure 1E). This migration from the fibrin polymer could also be seen *in vivo* (see below). Indeed, we used the mouse wounds to identify an ideal and suitable make up of the fibrin gel. The exact concentration of gel components was selected by titration to provide the most dilute system that would still form an adherent semi-solid. Eventually, using the mouse wounds for initial screening, the fibrin system we selected provided a minimum amount of fibrin so that cell migration and gel breakdown would be maximized. We found that a final dilution of less than 5mg/mL of fibrinogen and 25U/mL of fibrin was associated with a visible run-off of the sprayed solution, before a gel formed on contact with the wound. Therefore, this was used as the most suitable concentration of both fibrinogen and thrombin and for cell delivery. Increasing the concentration of thrombin, while keeping the fibrinogen concentration constant at 5mg/mL also led to a suitable gel. As explained above, the protease inhibitor aprotinin was not included in the spray system, since gel stability was not our goal and the purpose was to create a gel that would break down relatively quickly and release cells.

There is no single specific marker for MSC. Therefore, for flow cytometry and immunohistochemistry a panel of markers was chosen on the basis of published reports on human MSC. In agreement with these reports, the bone marrow-derived cells in the present study demonstrated the following characteristics and percentage positivity of surface markers: CD29 (99%), CD44 (99%), CD90 (81 %), CD105 (99%), CD166 (99%), CD34 (1.5%), and CD45 (<1%). Therefore, the cultured cells were virtually negative for CD34 and CD45. This overall flow cytometry profile was consistently present in cells through passages 4-8 and corresponded well with immunostaining of cells plated on glass slides, which was performed on cells in passages 4-7. The representative results for glass slides in Figure 2 were quite consistent with the flow cytometry studies, indicating positivity for MSC markers (CD29, CD44, CD90) and negativity for the hematopoietic marker CD34. It has been reported that the most primitive bone marrow stromal cells are CD34+.11 The cells we cultured were consistently CD34 negative. While this may indicate that our cultured bone marrow-derived cells may have had less "stemness," it also demonstrates that they were unlikely to be hematopoietic precursors. To further demonstrate the stem cell characteristics of the cultured cells, we performed functional assays to determine whether the cells could be induced to differentiate into osteocytes, adipocytes, and chondrocytes, which is an established characteristic of MSC. Results are shown in Figure 3, which for a total of four representative cell strains in passages 3-9 confirmed the conversion of the MSC to a phenotype of calcium production (Figure 3A), adipocyte and fat staining (Figure 3B), and cartilage formation (Figures 3C- E). It should be noted that in Figure 3A, negative control refers to human dermal fibroblasts, while positive control represents established and commercially available MSC.

### Example 10: Application of cultured MSC to human acute wounds

Having determined that bone marrow-derived cultured cells consistently display characteristics of MSC and could migrate from the fibrin matrix *in vitro* and in murine wounds, we next applied autologous cultured MSC to human wounds. We tested this approach with a fibrin spray delivery of the cells to both acute (n = 5) and chronic nonhealing wounds (n = 8). For the acute wounds, one subject had her bone marrow aspirated but later declined to participate in the study. Therefore, a total of four patients with postsurgical acute wounds were treated. As per protocol, the acute wounds consisted of defects left by removal of skin cancers (basal cell and squamous cell carcinomas) and that would not be ideally suited for primary closure or flaps. Patients had their bone marrow aspirated approximately 2 weeks before the surgery to allow for proper *in vitro* establishment of MSC cultures by the day of surgery. The cells were applied immediately after the removal of the skin cancer by Mohs surgery, an established procedure that helps insure complete cancer removal and tumor-free margins. Therefore, the applied cells were in their first 2-4 *in vitro* passages at the first application. Up to three applications were performed during the clinical course of the wound and, as stated in the Materials and Methods section, cells were not used beyond the 10th *in vitro* passage. The total fibrin volume (fibrinogen and an equal volume of thrombin) was no greater than 2 mL, and the same applies to the treatment of chronic wounds (see below). These postsurgical acute wounds received approximately 2x10⁶ cells/cm². Figure 4 illustrates this general approach of MSC application in a representative acute wound (subject #1 of Figure 5). Figure 4A shows the double-barreled syringe used to load the cells in the fibrinogen fraction of one barrel and the thrombin solution in the other. A gentle push on the common plunger while activating the CO₂ gas (2.5-5.0 psi) flowing through the tip of the syringe allowed an even mist of mixed fibrinogen and thrombin to reach the wound as a fibrin spray (Figure 4B). The syringe was held approximately 1-3 cm away from the wound bed, and the delivered spray polymerized very quickly to a gel consistency adhering to the wound bed. Indeed, as Figure 4B shows, the spray could be delivered to the wound in an upright position, and still without run-off of the spray or of the formed gel. Generally, the syringe was held at 45° to 60° from the wound bed and pointing from the edge toward the center of the wound. We found that the use of CO₂ flows greater than 5 psi would cause too forceful a spray and would result in splashing of considerable amounts of the gel outside the wound area.

Healing of the wound following a total of three applications per patient at least 1 week apart occurred uneventfully and by no later than week 8 (Figure 5). First, the wound bed filled with granulation tissue by 2 weeks. Pain relief was considerable, practically disappearing with the cell-based application. By 6 weeks, the large full-thickness wound almost completely resurfaced (Figure 4C) and healed completely a week later. Figure 4D shows that complete wound closure was durable, and that the wound remained healed at week 12. Follow-up of patients has continued to show persistent wound closure by 4-12 months after the procedure. Although the study did not have effectiveness as a primary outcome, some interesting observations emerged. Two of the four subjects enrolled had more than one wound, thus allowing the use of cell-containing fibrin in one wound and fibrin alone in the other. Figure 5A shows the healing trajectory in these four patients. One patient (#3) had two wounds, while another (#4) had three wounds. In each of these two subjects, one of the wounds was treated with MSC in fibrin, while the other(s) was treated with the fibrin spray alone. No delay in healing was observed with the use of cells in these two subjects. All wounds healed completely between weeks 7 and 8 after the surgery. Interestingly, however, the one wound that was dramatically larger at baseline, right after surgery (Figure 5A, subject #1, also shown in Figure 4), healed more rapidly than the smaller wounds and by week 7. This finding suggests that in acute wounds, which generally heal uneventfully, MSC application could lead to more accelerated resurfacing.

In the subjects with the acute wounds described above, biopsies of the wound bed were obtained at day 8 after the application of cultured cells. Using sequential and adjacent histological sections, we then determined whether immunostaining for specific markers could help support the hypothesis that the cultured cells had indeed migrated from the fibrin and were present in the superficial layers of the wound. Figure 6 shows representative immunostaining results using antibodies to CD29, CD45, and prolyl hydroxylase, a specific human fibroblast marker, in a subject who received either fibrin plus cells (left two panels) or fibrin alone (right panel). Labels "a," "b," and "c" in Figure 6 refer to magnifications of 4x, 10x, and 2x, respectively. CD29 is one of the markers for mesenchymal type of cells. We found CD29 immunostaining in the upper levels of the wound bed, and unassociated with immunostaining for CD45, the leukocyte common antigen and a marker that was not present in our cultured cells (see previous para- graph). Similar spindle cells in the upper layer of the wound bed also stained with the prolyl hydroxylase fibroblast marker (FibM). Taken together, these results suggest that at least some of the cultured cells may have migrated into the upper layers of the wound bed and possibly may have differentiated into a fibroblast phenotype. In contrast, as shown in the right panel ("c") of Figure 6 for the wound in the same subject treated with fibrin alone, the upper layers of the wound bed do not contain a substantial density of CD29+ cells. Indeed, there is a deeper infiltrate that appears to be CD29 and FibM positive and probably represents endogenous cells that are participating in the healing process. We also determined the possibility of tissue regeneration by focusing on the deposition of new elastic fibers. Since these wounds were full-thickness, one would not expect the presence of elastic fibers in the upper wound bed. However, as shown in Figure 7, definite elastic fibers were present when looked for both by the traditional Verhoeff-van Gieson special stain and by immunostaining with a specific antibody directed against human elastin. Comparable immunostaining for elastin was not observed in control biopsies (not shown).

### Example 11: Application of cultured MSC to human chronic wounds

Chronic wounds are very difficult to heal. In the context of studying the feasibility of our experimental approach in humans, we applied cultured MSC from autologous bone marrow to wounds of more than 1 year duration in the leg and foot of eight subjects that had not healed with a number of therapeutic approaches, including standard care, topically applied PDGF-BB, and living bioengineered skin constructs. These chronic wounds were due to venous insufficiency or diabetic neuropathy, but there was no evidence of significant arterial insufficiency. As with acute wounds, the patients were treated with MSC delivered topically in a fibrin spray, using the same methodology with respect to fibrinogen and thrombin concentrations and total volume of fibrin (no more than 2 mL). Because chronic wounds show considerable variation in baseline size, we tracked very carefully the number of cells delivered per cm² of wound surface, which was measured by computerized planimetry. A total of six subjects with chronic wounds were evaluated from the eight enrolled; two had to be excluded. One excluded subject was a woman with mild mental retardation who was found to chronically manipulate her foot wound. The other excluded subject was a man who was concomitantly found to have a systemic malignancy and thus could no longer continue to be in the protocol. No safety problems occurred during bone marrow aspiration and the application of cultured MSC in a fibrin spray. As with acute wounds, the protocol called for up to three applications of the stem cells. Figure 5B shows the healing trajectory of the six patients, and indicates a trend toward a decrease in ulcer size or complete wound closure by 16-20 weeks.

Figure 8 shows the example of one woman with a non-healing venous ulcer of the ankle, complicated by rheumatoid arthritis, which achieved complete wound closure using this approach. Her wound had not healed in over 10 years. Of the remaining five subjects, a mean wound area reduction of 40% was found in four, while one subject showed no change in the size of his wound. No adverse events were noted. Of great importance is the healing response within the first 2-4 weeks after each application of MSC in the subjects with chronic wounds. We analyzed this by determining the number of cells applied per cm² of total wound surface, as measured by computerized planimetry.

The graph in Figure 9 represents the correlation between the number of MSC applied to chronic wounds and the percent change in ulcer area at 2-4 weeks after each application (n = 17 data points). There was a very strong correlation indicating that, the greater the number of applied cells, the larger the reduction in ulcer area. Thus, using Spearmen Rank Correlation, we found r = -0.6389 (corrected for ties) with a 95% confidence interval of -0.8606 to-0.2135; p = 0.0058. As also suggested by the data points in Figure 9, additional statistical evaluation showed that only applications of greater than 1x10⁶ cells/cm² of the wound were associated with a subsequent (2-4 weeks) decrease in ulcer size of at least 10% (Fisher's Exact Test two-sided; p = 0.0345). One of the treated subjects had bilateral plantar ulcers from diabetes, and had his wounds treated with either the fibrin spray alone or cultured cells in a fibrin spray. Biopsies were taken from each of his plantar wounds, and sequential adjacent sections were analyzed by immunostaining.

Figure 10 shows that there was minimal or no overlap between CD45 and CD29 immunostaining in the wound bed of the MSC-treated wound. Interestingly, CD29 immunostaining was dramatically present in the MSC-treated wound but not in the one treated with fibrin alone. The dermal cells were spindle shaped, a pattern that was also observed when sequential sections were immunostained with antibodies to a specific fibroblast marker (Figure 10). These results, as with the acute wounds, suggest that the cells delivered to the wound were able to migrate from the fibrin matrix and had a mesenchymal phenotypic morphology.

### Example 12: Use of autologous MSC in full-thickness murine wounds

Tracking of MSC in humans is obviously difficult because of our inability to safely and reliably label cells prior to application. Moreover, we could only biopsy the human wounds early on after MSC application, and not after complete closure had occurred. Therefore, we next used mouse models of wounding to determine whether MSC would persist in the healed wound and lead to the formation of new structures, and whether a biological effect could be demonstrated in animals, such as the db/db diabetic mouse, which are known to heal with more difficulty. We approached this problem in the mouse in two different ways. Autologous bone marrow-derived cultured MSC were labeled with a red fluorescence dye (see Materials and Methods) and also used GFP+ MSC.

Figures 11A and B show the histological hematoxylin and eosin appearance and the red fluorescence of adjacent tissue sections 5 days after the application of red fluorescently labeled MSC to wounds made on the back of C57BL/6 mice. As the figures indicate, the labeled cells are able to penetrate into the wound bed. It should be noted that some of these studies were done just prior to the application of MSC to human wounds, and actually were instrumental in determining the proper application technique and concentrations of fibrinogen and thrombin for the delivery of cells in a spray system (see first part of Results section).

However, wounds made in the back of mice have the drawback that they heal mostly by contraction and a large dead space makes it difficult to know for certain whether the applied cells will remain in place. Therefore, we turned to a full-thickness model we recently established and that is now being used by other investigators also. This model consists of creating full-thickness wounds, down to fascia, on the dorsal aspect of the tail, approximately 1 cm distal to the body. These excisions take up to 3 weeks to heal in normal mice, and reflect resurfacing by epithelial migration rather than by contraction.

Using this model in db/db mice and their control (db/+) littermates, we delivered a single application of either fibrin alone or fibrin containing autologous bone marrow-derived cultured MSC immediately after wounding. As with human MSC, we determined mouse cluster designation markers by flow cytometry and immunohistochemistry. The applied cells had the following profile: CD29+, CD44+, SCA-1+, CD34-, CD45/LCA-, CD31-. Figure 11C shows the results in tail wounds in db/db mice and their control littermates treated with either fibrin alone orMSC-containing fibrin.We used the Bonferroni Multiple Comparisons Test to determine the significance of the results. By day 10 after wounding, the control mice showed accelerated healing with MSC application (p<0.01) compared to fibrin alone, while a strong trend but no statistically significant difference was detected in the db/db mice. By day 20 after wounding, MSC application led to a statistically significant difference from fibrin alone in the db/db mice (p<0.001), and this difference continued to be present by day 25 ( p<0.05). The application of fibrin alone did not stimulate healing when compared to air-exposed wounds (p>0.05, not shown).

In other mouse tail-wound experiments designed to track the fate of topically delivered cells, we applied mouse autologous GFP+ MSC immediately after tail wounding and analyzed frozen sections from the wound site for the presence of green fluorescence. Because of the very friable nature of the wounded site at early time points, which made it difficult to obtain intact sections, we took shave biopsies for frozen sections at later times and immediately after wound closure. For imaging, we used different filters capable of detecting false green fluorescence positivity from GFP (see legend to Figure 12). Thus, we used filters for FITC (510-560 nm), light microscopy, Texas Red (645/ 75 nm), and DAPI (435-475 nm). Using these filters, we were also able to construct composite photomicrographs, again to confirm the specificity of any green fluorescence. We found that, by day 18 after wounding, definite clusters of GFP+ cells could no longer be demonstrated at the wound site, and only rare individual GFP+ cells, not due to auto-fluorescence, could be detected. Similarly, keratinocytes were not GFP+. Interestingly, however, we uncommonly found stable endothelial structures that were clearly GFP+ (approximately one per 20 high-power fields).

The representative photomicrographs in Figure 12, using different filters and overlay of images to eliminate false positivity, indicate a blood vessel that shows definite green fluorescence. These results suggest that the applied cells, at least those that are GFP+, may not persist in great numbers in the healed wound. The acceleration of healing clearly present with the application of MSC to db/db mice wounds indicates that the MSC may play a stimulatory role in spite of the lack of long-term persistence.

### References:

1. Weiss, E., Yamaguchi, Y., Falabella, A., Crane, S., Tokuda, Y., and Falanga, V. Uncross-linked fibrin substrates inhibit keratinocyte spreading and replication: correction with fibronectin and factorXIII cross-linking. J Cell Physiol 174(1): 58-65, 1998.
2. Ho, W., Tawil, B., Dunn, J.C., and Wu, B.M. The behavior of human mesenchymal stem cells in 3D fibrin clots: dependence on fibrinogen concentration and clot structure. Tissue Eng 12(6): 1587-95, 2006.
3. Becker, J.C., Domschke, W., and Pohle, T. Biological in vitro effects of fibrin glue: fibroblast proliferation, expression and binding of growth factors. Scand J Gastroenterol 39(10): 927-32, 2004.
4. Catelas, I., Sese, N., Wu, B.M., Dunn, J.C., Helgerson, S., and Tawil, B. Human mesenchymal stem cell proliferation and osteogenic differentiation in fibrin gels in vitro. Tissue Eng 12: 2385-2396, 2006.
5. Cox, S., Cole, M., and Tawil, B. Behavior of human dermal fibroblasts in three-dimensional fibrin clots: dependence on fibrinogen and thrombin concentration. Tissue Eng 10(5-6): 942-54, 2004.

## Claims

1. A fibrin sealant composition comprising a fibrinogen complex (FC) component, a thrombin component, and a cellular component, wherein the concentration of fibrinogen used to form the gel is between from 2 to 5 mg/ml, wherein the cellular component comprises one or more of stem/progenitor cells, and wherein the fibrin sealant comprises at least 1.5 - 2.0 X 10⁶ stem/progenitor cells / cm².

2. The fibrin sealant composition of claim 1, wherein the stem/progenitor cells are CD34⁺/lin⁻/CD45⁻ or Sca-1⁺/lin⁻/CD45⁻ very small embryonic like (VSEL) stem cells.

3. The fibrin sealant composition of claim 1, stem/progenitor cells are selected from the group consisting of bone marrow derived cells, hematopoietic stem cells, mesenchymal stem cells, peripheral blood stem cells, and mixtures and combinations thereof.

4. The fibrin sealant composition of claim 1, wherein the stem/progenitor cells are bone marrow derived cells.

5. The fibrin sealant composition of claim 1, wherein the stem/progenitor cells are peripheral blood stem cells.

6. A fibrin sealant composition of claim 1 for use in therapy, comprising: a) combining stem/progenitor cells with a fibrin sealant to form a wound sealant, said fibrin sealant comprising calcic thrombin and fibrinogen, wherein the concentration of calcic thrombin is 25 U/ml; b) administering the fibrin sealant composition to a cutaneous wound, wherein the fibrin sealant composition is administered in the form of a polymerized gel or spray.

7. The fibrin sealant composition for use as in claim 6, wherein the therapy is for use in ameliorating the formation of scars at a wound site.

8. The fibrin sealant composition for use as in claim 6, wherein the therapy is for use in treating scleroderma.

9. The fibrin sealant composition for use as in any one of claims 6, 7 or 8, wherein the stem/progenitor cells are CD34⁺/lin⁻/CD45⁻ or Sca-1⁺/lin⁻/CD45⁻ VSEL stem cells.

10. The fibrin sealant composition for use as in any one of claims 6, 7 or 8, wherein the fibrin sealant is administered to the site of the wound at least two times over the span of three weeks.

11. The fibrin sealant composition for use as in any one of claims 6, 7 or 8, wherein the stem/ progenitor cells are bone marrow derived cells, hematopoietic stem cells, mesenchymal stem cells, peripheral blood stem cells, and mixtures and combinations thereof.

12. The fibrin sealant composition for use as in any of claims 6, 7 or 8, wherein the fibrin sealant is administered to the site of the wound in the form of a spray at a CO₂ pressure of less than 34 475 Pa (N/m²) (5 psi).

13. The fibrin sealant composition for use as in any of claims 6, 7 or 8 wherein the fibrin sealant is topically applied to the site of the wound; and optionally wherein a skin substitute is applied to the site of the wound.

14. The fibrin sealant composition for use as in claim 7, wherein the wound is the result of a burn injury.

15. The fibrin sealant composition for use as in claim 7, wherein the scars are hypertrophic scars.

16. A method of making a fibrin sealant composition comprising: providing a fibrinogen complex (FC) component, a calcic thrombin component, and a cellular component; adding the cellular component to the FC component before admixture of the FC component with the calcic thrombin component; and adding the calcic thrombin component to the combined FC/cellular component mixture, wherein the concentration of fibrinogen is from 2 to 5 mg/ml, and wherein the final concentration of stem/progenitor cells is at least from 1.5 to 2.0 X 10⁶ stem/progenitor cells / cm².

17. The method of claim 16, wherein the stem/progenitor cells are CD34⁺/lin⁻/CD45⁻ or Sca-1⁺/lin⁻/CD45⁻VSEL stem cells.

18. The method of claim 16, wherein the stem/progenitor cells are selected from the group consisting of bone marrow derived cells, hematopoietic stem cells, mesenchymal stem cells, peripheral blood stem cells, and mixtures and combinations thereof.

19. A kit for preparing the fibrin sealant composition of claim 1 comprising, a) a first vial or first storage container containing the fibrinogen complex component, and wherein said first vial or first storage container optionally comprises a cellular component, and b) a second vial or second storage container having a thrombin component, said kit further containing instructions for use thereof, wherein said kit optionally contains a third vial or third storage container having a cellular component when said first vial or first storage container does not include a cellular component.

20. The method of claim 16 or the kit of 19, wherein the concentration of thrombin is 25 U/ml.

21. The kit of claim 19, wherein said kit further comprises reagents for characterizing the cellular component.

22. The fibrin sealant composition of claim 1, wherein the concentration of fibrinogen used to form the gel is 5 mg/ml.

23. The fibrin sealant composition for use as in claim 6, wherein the concentration of fibrinogen used to form the fibrin sealant composition is 5 mg/ml.

24. The method of claim 16 or the kit of claim 19, wherein the concentration of fibrinogen is 5 mg/ml.

## Patentansprüche

1. Fibrinversiegelungszusammensetzung umfassend eine Fibrinogenkomplex (fibrinogen complex, FC)-Komponente, eine Thrombinkomponente, und eine zelluläre Komponente, wobei die Konzentration von Fibrinogen, das verwendet wird, um das Gel zu bilden, zwischen 2 und 5 mg/ml beträgt,
wobei die zelluläre Komponente einen oder mehrere aus Stamm-/Vorläuferzellen umfasst, und wobei die Fibrinversiegelung wenigstens 1,5 - 2,0 x 10⁶ Stamm-/Vorläuferzellen / cm² umfasst.

2. Fibrinversiegelungszusammensetzung nach Anspruch 1, wobei die Stamm-/Vorläuferzellen CD34⁺/lin⁻/CD45⁻ oder Sca-1⁺/lin⁻/CD45⁻, sehr kleine embryonal ähnliche (VSEL) Stammzellen sind.

3. Fibrinversiegelungszusammensetzung nach Anspruch 1, wobei die Stamm-/Vorläuferzellen ausgewählt sind aus der Gruppe bestehend aus von Knochenmark abstammenden Zellen, hämatopoetischen Stammzellen, mesenchymalen Stammzellen, periphereren Blutstammzellen sowie Mischungen und Kombinationen derselben.

4. Fibrinversiegelungszusammensetzung nach Anspruch 1, wobei die Stamm-/Vorläuferzellen von Knochenmark abstammende Zellen sind.

5. Fibrinversiegelungszusammensetzung nach Anspruch 1, wobei die Stamm-/Vorläuferzellen periphere Blutstammzellen sind.

6. Fibrinversiegelungszusammensetzung nach Anspruch 1 zur Verwendung bei der Therapie, umfassend: a) das Kombinieren von Stamm-/Vorläuferzellen mit einer Fibrinversiegelung zur Bildung einer Wundversiegelung, wobei die Fibrinversiegelung kalziumhaltiges Thrombin und Fibrinogen umfasst, wobei die Konzentration von kalziumhaltigem Thrombin 25 U/ml beträgt; b) Verabreichung der Fibrinversiegelungszusammensetzung auf einer kutanen Wunde, wobei die Fibrinversiegelungszusammensetzung in Form eines polymerisierten Gels oder Sprays verabreicht wird.

7. Fibrinversiegelungszusammensetzung zur Verwendung nach Anspruch 6, wobei die Therapie in der Verwendung zur Linderung der Bildung von Narben an der Wundstelle liegt.

8. Fibrinversiegelungszusammensetzung zur Verwendung nach Anspruch 6, wobei die Therapie in der Verwendung zur Behandlung von Sklerodermie liegt.

9. Fibrinversiegelungszusammensetzung zur Verwendung nach einem der Ansprüche 6, 7, oder 8, wobei die Stamm-/Vorläuferzellen CD34⁺/lin⁻/CD45⁻ oder Sca-1⁺/lin⁻/CD45⁻VSEL-Stammzellen sind.

10. Fibrinversiegelungszusammensetzung nach einem der Ansprüche 6, 7, oder 8, wobei die Fibrinversiegelung der Wundstelle mindestens zwei Mal über den Zeitraum von drei Wochen verabreicht wird.

11. Fibrinversiegelungszusammensetzung zur Verwendung nach einem der Ansprüche 6, 7 oder 8, wobei die Stamm-/Vorläuferzellen von Knochenmark abstammende Zellen, hämatopoetische Stammzellen, mesenchymale Stammzellen, peripherere Blutstammzellen sowie Mischungen und Kombinationen derselben sind.

12. Fibrinversiegelungszusammensetzung zur Verwendung nach einem der Ansprüche 6, 7 oder 8, wobei die Fibrinversiegelung der Wundstelle in Form eines Sprays unter einem CO₂-Druck von weniger als 34 475 Pa (N/m²) (5 psi) verabreicht wird.

13. Fibrinversiegelungszusammensetzung zur Verwendung nach einem der Ansprüche 6, 7 oder 8, wobei die Fibrinversiegelung topisch auf die Wundstelle aufgebracht wird; und optional wobei ein Hautersatz auf der Wundstelle angewendet wird.

14. Fibrinversiegelungszusammensetzung zur Verwendung nach Anspruch 7, wobei die Wunde das Ergebnis einer Verbrennungsverletzung ist.

15. Fibrinversiegelungszusammensetzung zur Verwendung nach Anspruch 7, wobei die Narben hypertrophe Narben sind.

16. Verfahren zur Herstellung einer Fibrinversiegelungszusammensetzung umfassend:
das zur Verfügung stellen einer Fibrinkomplex (FC)-Komponente, einer kalziumhaltigen Thrombinkomponente und einer zellulären Komponente; das Hinzufügen der zellulären Komponente zur FC-Komponente vor dem Vermischen der FC-Komponente mit der kalziumhaltigen Thrombinkomponente; und Hinzufügen der kalziumhaltigen Thrombinkomponente zur kombinierten FC/zellulären Komponente, wobei die Konzentration von Fibrinogen zwischen 2 und 5 mg/ml beträgt, und wobei die Endkonzentration der Stamm-/Vorläuferzellen wenigstens 1,5 - 2,0 x 10⁶ Stamm-/Vorläuferzellen / cm² beträgt.

17. Verfahren nach Anspruch 16, wobei die Stamm-/Vorläuferzellen CD34⁺/lin⁻/CD45⁻oder Sca-1⁺/lin⁻/CD45-VSEL-Stammzellen sind.

18. Verfahren nach Anspruch 16, wobei die Stamm-/Vorläuferzellen ausgewählt sind aus der Gruppe bestehend aus von Knochenmark abstammenden Zellen, hämatopoetischen Stammzellen, mesenchymalen Stammzellen, periphereren Blutstammzellen sowie Mischungen und Kombinationen derselben.

19. Kit zur Herstellung der Fibrinversiegelungszusammensetzung nach Anspruch 1, umfassend, a) eine erste Phiole oder einen ersten Aufbewahrungsbehälter, welche/welcher die die Fibrinogenkomplexkomponente umfasst, und wobei die erste Phiole oder der erste Aufbewahrungsbehälter optional eine zelluläre Komponente umfasst, und b) eine zweite Phiole oder einen zweiten Aufbewahrungsbehälter mit einer Thrombinkomponente, wobei das Kit ferner Instruktionen zur Verwendung dieser umfasst, wobei das Kit optional eine dritte Phiole oder einen dritten Aufbewahrungsbehälter umfasst, mit einer zellulären Komponente, wenn die erste Phiole oder der erste Aufbewahrungsbehälter keine zelluläre Komponente enthält.

20. Verfahren nach Anspruch 16 oder Kit nach Anspruch 19, wobei die Konzentration von Thrombin 25 U/ml beträgt.

21. Kit nach Anspruch 19, wobei das Kit ferner Reagenzien für die Charakterisierung der zellulären Komponente umfasst.

22. Fibrinversiegelungszusammensetzung nach Anspruch 1, wobei die Konzentration von Fibrinogen, das verwendet wird, um das Gel zu bilden, 5 mg/ml beträgt.

23. Fibrinversiegelungszusammensetzung zur Verwendung nach Anspruch 6, wobei die die Konzentration von Fibrinogen, das verwendet wird, um die Fibrinversiegelungszusammensetzung zu bilden, 5 mg/ml beträgt.

24. Verfahren nach Anspruch 16 oder Kit nach Anspruch 19, wobei die Konzentration von Fibrinogen, das verwendet wird, 5 mg/ml beträgt.

## Revendications

1. Composition de colle de fibrine comprenant un composant complexe fibrinogène (FC), un composant thrombine, et un composant cellulaire, dans laquelle la concentration en fibrinogène utilisée pour former le gel est comprise entre 2 et 5 mg/mL,
dans laquelle le composant cellulaire comprend une ou plusieurs cellules souches/progénitrices, et dans laquelle la colle de fibrine comprend au moins 1,5 à 2,0 x 10⁶ cellules souches/progénitrices / cm².

2. Composition de colle de fibrine selon la revendication 1, dans laquelle les cellules souches/progénitrices sont de très petites cellules souches de type embryonnaires (VSEL) CD34⁺/lin⁻/CD45⁻ ou Sca-1⁺/lin⁻/CD45⁻.

3. Composition de colle de fibrine selon la revendication 1, dans laquelle les cellules souches/progénitrices sont sélectionnées dans le groupe constitué de cellules dérivées de moelle osseuse, de cellules souches hématopoïétiques, de cellules souches mésenchymateuses, de cellules souches du sang périphérique, et de mélanges et combinaisons de celles-ci.

4. Composition de colle de fibrine selon la revendication 1, dans laquelle les cellules souches/progénitrices sont des cellules dérivées de moelle osseuse.

5. Composition de colle de fibrine selon la revendication 1, dans laquelle les cellules souches/progénitrices sont des cellules souches de sang périphérique.

6. Composition de colle de fibrine selon la revendication 1, pour son utilisation en thérapie, ladite composition comprenant : a) la combinaison de cellules souches/progénitrices avec une colle de fibrine pour former une colle pour plaies, ladite colle de fibrine comprenant de la thrombine calcique et du fibrinogène, dans laquelle la concentration en thrombine calcique est de 25 U/mL ; b) l'administration de la composition de colle de fibrine à une plaie cutanée, dans laquelle la composition de colle de fibrine est administrée sous la forme d'une pulvérisation ou d'un gel polymérisé.

7. Composition de colle de fibrine pour son utilisation selon la revendication 6, dans laquelle la thérapie est destinée à être utilisée pour améliorer la formation de cicatrices au niveau d'un site de plaie.

8. Composition de colle de fibrine pour son utilisation selon la revendication 6, dans laquelle la thérapie est destinée à être utilisée dans le traitement de la sclérodermie.

9. Composition de colle de fibrine pour son utilisation selon l'une quelconque des revendications 6, 7 ou 8, dans laquelle les cellules souches/progénitrices sont des cellules souches VSEL CD34⁺/lin⁻/CD45⁻ ou Sca-1⁺/lin⁻/CD45⁻.

10. Composition de colle de fibrine pour son utilisation selon l'une quelconque des revendications 6, 7 ou 8, dans laquelle la colle de fibrine est administrée au site de la plaie au moins deux fois sur une durée de trois semaines.

11. Composition de colle de fibrine pour son utilisation selon l'une quelconque des revendications 6, 7 ou 8, dans laquelle les cellules souches/progénitrices sont des cellules dérivées de moelle osseuse, des cellules souches hématopoïétiques, des cellules souches mésenchymateuses, des cellules souches du sang périphérique, et des mélanges et combinaisons de celles-ci.

12. Composition de colle de fibrine pour son utilisation selon l'une quelconque des revendications 6, 7 ou 8, dans laquelle la colle de fibrine est administrée au site de la plaie sous la forme d'une pulvérisation à une pression de CO₂ inférieure à 34 475 Pa (N/m²) (5 psi).

13. Composition de colle de fibrine pour son utilisation selon l'une quelconque des revendications 6, 7 ou 8, dans laquelle la colle de fibrine est appliquée au site de la plaie par voie topique ; et facultativement dans laquelle un substitut de peau est appliqué au site de la plaie.

14. Composition de colle de fibrine pour son utilisation selon la revendication 7, dans laquelle la plaie est le résultat d'une lésion par brûlure.

15. Composition de colle de fibrine pour son utilisation selon la revendication 7, dans laquelle les cicatrices sont des cicatrices hypertrophiques.

16. Procédé de fabrication d'une composition de colle de fibrine, le procédé comprenant : la fourniture d'un composant complexe fibrinogène (FC), d'un composant thrombine calcique, et d'un composant cellulaire ; l'ajout du composant cellulaire au composant FC avant le mélange du composant FC avec le composant thrombine calcique ; et l'ajout du composant thrombine calcique au mélange combiné FC/composant cellulaire, dans lequel la concentration en fibrinogène est de 2 à 5 mg/mL, et dans lequel la concentration finale en cellules souches/progénitrices est d'au moins 1,5 à 2,0 x 10⁶ cellules souches/progénitrices / cm².

17. Procédé selon la revendication 16, dans lequel les cellules souches/progénitrices sont des cellules souches VSEL CD34⁺/lin⁻/CD45⁻ ou Sca-1⁺/lin⁻/CD45⁻.

18. Procédé selon la revendication 16, dans lequel les cellules souches/progénitrices sont sélectionnées dans le groupe constitué de cellules dérivées de moelle osseuse, de cellules souches hématopoïétiques, de cellules souches mésenchymateuses, de cellules souches du sang périphérique, et de mélanges et combinaisons de celles-ci.

19. Kit de préparation de la composition de colle de fibrine selon la revendication 1, ledit kit comprenant : a) un premier flacon ou premier récipient de stockage contenant le composant complexe fibrinogène, et dans lequel ledit premier flacon ou premier récipient de stockage comprend facultativement un composant cellulaire, et b) un deuxième flacon ou deuxième récipient de stockage ayant un composant thrombine, ledit kit contenant en outre des instructions pour son utilisation, dans lequel ledit kit contient facultativement un troisième flacon ou un troisième récipient de stockage ayant un composant cellulaire lorsque ledit premier flacon ou ledit premier récipient de stockage ne comprend pas de composant cellulaire.

20. Procédé selon la revendication 16 ou kit selon la revendication 19, dans lesquels la concentration en thrombine est de 25 U/mL.

21. Kit selon la revendication 19, dans lequel ledit kit comprend en outre des réactifs de caractérisation du composant cellulaire.

22. Composition de colle de fibrine selon la revendication 1, dans laquelle la concentration en fibrinogène utilisée pour former le gel est de 5 mg/mL.

23. Composition de colle de fibrine pour son utilisation selon la revendication 6, dans laquelle la concentration en fibrinogène utilisée pour former la composition de colle de fibrine est de 5 mg/mL.

24. Procédé selon la revendication 16 ou kit selon la revendication 19, dans lesquels la concentration en fibrinogène est de 5 mg/mL.
